(19) Europäisches Patentamt
European Patent Office
Office européen des brevets

(11) Veröffentlichungsnummer: **0 402 716 A1**

# (12) EUROPÄISCHE PATENTANMELDUNG

(21) Anmeldenummer: **90110451.3**

(22) Anmeldetag: **01.06.90**

(51) Int. Cl.5: **C07D 277/58, C07D 277/587, C07D 417/12, A01N 43/78**

(30) Priorität: **14.06.89 DE 3919365**

(43) Veröffentlichungstag der Anmeldung: **19.12.90 Patentblatt 90/51**

(84) Benannte Vertragsstaaten: **BE CH DE FR GB IT LI NL**

(71) Anmelder: **BAYER AG**

**D-5090 Leverkusen 1 Bayerwerk(DE)**

(72) Erfinder: **Beck, Gunther, Dr.**
**Am Mittelberg 19**
**D-5090 Leverkusen 1(DE)**
Erfinder: **Dutzmann, Stefan, Dr.**
**Leinenweberweg 33**
**D-4000 Düsseldorf 13(DE)**
Erfinder: **Brandes, Wilhelm, Dr..**
**Eichendorffstrasse 3**
**D-5653 Leichlingen(DE)**
Erfinder: **Paulus, Wilfried, Dr.**
**Deswatinestrasse 90**
**D-4150 Krefeld(DE)**

(54) **2-Acylamino-4-halogen-5-nitrothiazol-Derivate, Verfahren zu ihrer Herstellung, ihre Verwendung in Schädlingsbekämpfungsmitteln und neue Zwischenprodukte.**

(57) Die Verbindungen der Formel (I)

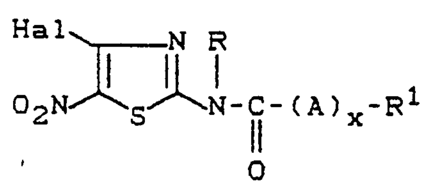

(I)

in welcher
Hal, R, $R^1$, A und x die in der Beschreibung gegebene Bedeutung haben, und ihre Verwendung zur Bekämpfung von Schädlingen.

Die neuen Verbindungen sind durch die Formel (I) definiert und können nach Analogieverfahren hergestellt werden Z.B. kann man geeignete 2,4-Dihalogen-5-nitrothiazole mit geeigneten Nucleophilen umsetzen oder geeignete 2-Amino-4-halogen-5-nitro-thiazol-Derivate mit geeigneten Halogencarbonyl-Verbindungen bzw. Carbonsäureanhydriden bzw. Isocyanaten umsetzen oder geeignete 4-Halogen-2-halogencarbonylamino-5-nitro-thiazol-Derivate, die ebenfalls neu sind, mit geeigneten Nucleophilen umsetzen. Auch die neuen Zwischenprodukte können nach Analogieverfahren hergestellt werden.

EP 0 402 716 A1

## 2-Acylamino-4-halogen-5-nitrothiazol-Derivate, Verfahren zu ihrer Herstellung, ihre Verwendung in Schädlingsbekämpfungsmitteln und neue Zwischenprodukte

Die vorliegende Erfindung betrifft neue 2-Acylamino-4-halogen-5-nitrothiazol-Derivate. mehrere Verfahren zu ihrer Herstellung sowie ihre Verwendung in Schädlingsbekämpfungsmitteln und einige neue Zwischenprodukte.

Es ist bekannt, daß Thiazole, die z.B. durch Hydroxy-phenoxy substituiert sind, in der Medizin Verwendung finden zur Behandlung von Tumoren (vgl. WO 88/00944).

Weiterhin ist bekannt, daß 2.4-Dichlor-5-nitro-thiazol eine mikrobizide, vor allem eine fungizide Wirkung im Pflanzenschutz aufweist (vgl. DE-OS 3 518 520).

Auch sind Thiazol-Derivate, wie z.B. 4,5-Dichlor-2-propinyloxy-thiazol, als Synergisten bekannt (vgl. DE-OS 3 030 661).

Weiterhin ist u.a. das 2-Acetamino-4-iodo-5-nitrothiazol bekannt als Ausgangsprodukt für 2-Amino-4-alkylsulfonyl-5-nitrothiazol-azo-Derivate, die als Färbemittel Verwendung finden (vgl. US 2 852 504 und US 2 839 523).

Weiterhin sind Phenoxycarbonsäureamide bekannt, die u.a. auch einen Thiazolrest enthalten können. Diese Verbindungen sind als Herbizide bekannt (vgl. DE-OS 3 101 889).

Weiterhin ist bekannt, daß bestimmte substituierte Thiazole, wie z.B. 4-Benzimidazol-2-yl-thiazol (Thiabendazol), als Fungizide im Materialschutz verwendet werden (vgl. z.B. R. Wegler "Chemie der Pflanzenschutz-und Schädlingsbekämpfungsmittel", Bd. 2, S. 124 und Bd. 3, S. 292; Springer Verlag, Berlin, Heidelberg, N.Y. 1970).

Das Wirkungsspektrum dieser vorbekannten Verbindungen ist jedoch lückenhaft und ihre mikrobizide Wirksamkeit ist in bestimmten Indikationsgebieten nicht immer befriedigend.

Es wurden neue 2-Acylamino-4-halogen-5-nitrothiazol-Derivate der Formel (I)

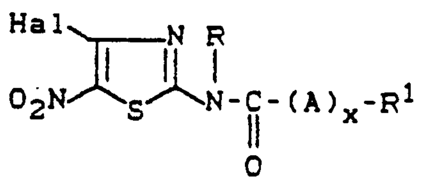

(I)

in welcher

Hal für Halogen steht,

x für eine ganze Zahl 0 oder 1 steht,

A für O, S oder N-$R^2$ steht, worin

$R^2$ für Wasserstoff, Alkyl, Alkenyl, Halogenalkyl, Cyanalkyl, Alkoxycarbonylalkyl oder für jeweils gegebenenfalls ein- bis mehrfach, gleich oder verschieden substituiertes Cycloalkyl, Aryl oder Aralkyl steht,

R für Wasserstoff, Alkyl, Alkenyl oder Alkinyl steht, wobei die vorgenannten Reste jeweils gegebenenfalls ein- bis mehrfach, gleich oder verschieden durch Halogen, Alkoxy, Aryloxy, Alkylmercapto, Arylmercapto und Cyano substituiert sein können, für gegebenenfalls ein- bis mehrfach, gleich oder verschieden durch Alkyl substituiertes Cycloalkyl steht, wobei zusätzlich zu der Alkylsubstitution ein Ring ankondensiert sein kann, für gegebenenfalls ein-bis mehrfach, gleich oder verschieden durch Halogen, Alkyl, Halogenalkyl, Nitro, Alkoxy, Alkylmercapto und Cyan im Arylteil substituiertes Aralkyl steht oder für gegebenenfalls ein- bis mehrfach, gleich oder verschieden durch Halogen, Alkyl, Alkenyl, Alkinyl, Halogenalkyl, Nitro, Alkoxy, Alkylmercapto, Dialkylamino, Carbalkoxy, Carbamoyl, N-Alkylcarbamoyl, N,N-Dialkylcarbamoyl, Halogenalkyloxy, Halogenalkylmercapto, Alkylsulfonylamino, Alkylsulfonyl, Aryl, Aryloxy, Arylmercapto, Acyl oxy, Acyl, Sulfamoyl, N-Alkylsulfamoyl, N-Acyl-N-alkylsulfamoyl, N,N-Dialkylsulfamoyl, Aralkyloxy, Aralkylmercapto, Acylamino, Acylaminosulfonyl, Acylalkylamino, Cycloalkyl und Cyan substituiertes Aryl steht und

R' für Wasserstoff, Alkyl, Alkenyl, Alkinyl, Halogenalkyl, Cyanalkyl, Alkoxycarbonylalkyl oder für jeweils gegebenenfalls ein- bis mehrfach, gleich oder verschieden substituiertes Cycloalkyl, Aryl Heteroaryl, Aralkyl oder Heteroaralkyl steht oder

R und R' gemeinsam mit der Gruppierung -N-CO-$(A)_x$-, an der sie stehen, einen Ring bilden, der gegebenenfalls durch ein oder mehrere weitere, gleiche oder verschiedene Heteroatome unterbrochen sein kann und der gegebenenfalls ein- bis mehrfach, gleich oder verschieden substituiert sein kann oder

$R^1$ und $R^2$ gemeinsam mit dem Stickstoffatom, an der sie stehen, einen Ring bilden, der gegebenenfalls durch ein oder mehrere weitere, gleiche oder verschiedene Heteroatome unterbrochen sein kann und der gegebenenfalls ein- bis mehrfach, gleich oder verschieden substituiert sein kann und gegebenenfalls weitere Ringe ankondensiert sein können,
mit der Ausnahme, daß $R^1$ nicht Wasserstoff ist, wenn A für O oder S steht und ausgenommen die Verbindung 2-Acetylamino-4-iodo-5-nitro-thiazol,
gefunden.

Weiterhin wurde gefunden, daß man die 2-Acylamino-4-halogen-5-nitrothiazol-Derivate der Formel (I)

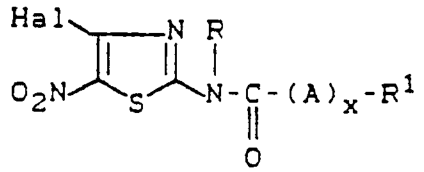

(I)

in welcher
Hal für Halogen steht,
x für eine ganze Zahl 0 oder 1 steht,
A für O, S oder N-$R^2$ steht, worin
$R^2$ für Wasserstoff, Alkyl, Alkenyl, Halogenalkyl, Cyanalkyl, Alkoxycarbonylalkyl oder für jeweils gegebenenfalls ein- bis mehrfach, gleich oder verschieden substituiertes Cycloalkyl, Aryl oder Aralkyl steht,
R für Wasserstoff, Alkyl, Alkenyl oder Alkinyl steht, wobei die vorgenannten Reste jeweils gegebenenfalls ein- bis mehrfach, gleich oder verschieden durch Halogen, Alkoxy, Aryloxy, Alkylmercapto, Arylmercapto und Cyano substituiert sein können, für gegebenenfalls ein- bis mehrfach, gleich oder verschieden durch Alkyl substituiertes Cycloalkyl steht, wobei zusätzlich zu der Alkylsubstitution ein Ring ankondensiert sein kann, für gegebenenfalls ein-bis mehrfach, gleich oder verschieden durch Halogen, Alkyl, Halogenalkyl, Nitro, Alkoxy, Alkylmercapto und Cyan im Arylteil substituiertes Aralkyl steht oder für gegebenenfalls ein- bis mehrfach, gleich oder verschieden durch Halogen, Alkyl, Alkenyl, Alkinyl, Halogenalkyl, Nitro, Alkoxy, Alkylmercapto, Dialkylamino, Carbalkoxy, Carbamoyl, N-Alkylcarbamoyl, N,N-Dialkylcarbamoyl, Halogenalkyloxy, Halogenalkylmercapto, Alkylsulfonylamino, Alkylsulfonyl, Aryl, Aryloxy, Arylmercapto, Acyloxy, Acyl, Sulfamoyl, N-Alkylsulfamoyl, N-Acyl-N-alkylsulfamoyl, N,N-Dialkylsulfamoyl, Aralkyloxy, Aralkylmercapto, Acylamino, Acylaminosulfonyl, Acylalkylamino, Cycloalkyl und Cyan substituiertes Aryl steht und
$R^1$ für Wasserstoff, Alkyl, Alkenyl, Alkinyl, Halogenalkyl, Cyanalkyl, Alkoxycarbonylalkyl oder für jeweils gegebenenfalls ein- bis mehrfach, gleich oder verschieden substituiertes Cycloalkyl, Aryl, Heteroaryl, Aralkyl oder Heteroaralkyl steht oder
R und $R^1$ gemeinsam mit der Gruppierung $-N-CO-(A)_x-$, an der sie stehen, einen Ring bilden, der gegebenenfalls durch ein oder mehrere weitere, gleiche oder verschiedene Heteroatome unterbrochen sein kann und der gegebenenfalls ein- bis mehrfach, gleich oder verschieden substituiert sein kann oder
$R^1$ und $R^2$ gemeinsam mit dem Stickstoffatom, an dem sie stehen, einen Ring bilden, der gegebenenfalls durch ein oder mehrere weitere, gleiche oder verschiedene Heteroatome unterbrochen sein kann und der gegebenenfalls ein- bis mehrfach, gleich oder verschieden substituiert sein kann und gegebenenfalls weitere Ringe ankondensiert sein können,
mit der Ausnahme, daß $R^1$ nicht Wasserstoff ist, wenn A für O oder S steht,
erhält, wenn man

a) 2,4-Dihalogen-5-nitrothiazole der Formel (II)

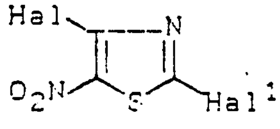

(II)

in welcher
$Hal^1$ für Halogen steht,
Hal die oben angegebene Bedeutung hat und
Hal und $Hal^1$ gleich oder verschieden sein können,

mit Nucleophilen der Formel (III)

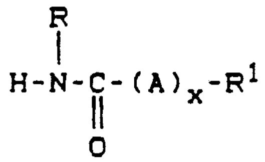

(III)

in welcher
A, x, R und $R^1$ die oben angegebene Bedeutung haben, oder deren Metallsalze gegebenenfalls in Gegenwart von Säurebindemitteln und in Gegenwart von Verdünnungsmitteln umsetzt.
oder

b) 2-Amino-4-halogen-5-nitrothiazol-Derivate der Formel (IV)

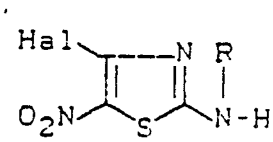

(IV)

in welcher
Hal und R die oben angegebenen Bedeutungen haben,
mit Halogencarbonyl-Verbindungen der Formel (V)

$$Hal^3-\underset{\underset{O}{\|}}{C}-(A)_x-R^1 \quad (V)$$

in welcher
$Hal^3$ für Halogen steht und
A, X und $R^1$ die oben angegebene Bedeutung haben,
gegebenenfalls in Gegenwart von Säurebindemitteln und in Gegenwart von Verdünnungsmitteln umsetzt.
oder

c) für den Fall, daß x für 0 steht und $R^1$ die oben angegebene Bedeutung ausgenommen Wasserstoff hat,
2-Amino-4-halogen-5-nitrothiazol-Derivate der Formel (IV) mit Carbonsäureanhydriden der Formel (VI)

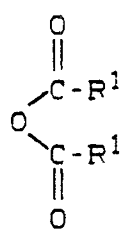

(VI)

in welcher
$R^1$ die oben angegebene Bedeutung ausgenommen Wasserstoff hat,
gegebenenfalls in Gegenwart von Verdünnungsmitteln umsetzt,
oder

d) für den Fall, daß x für 1 und A für NH steht, 2-Amino-4-halogen-5-nitrothiazol-Derivate der Formel (IV) mit Isocyanaten der Formel (VII)

$O=C=N-R^1$ (VII)

in welcher
$R^1$ die oben angegebene Bedeutung ausgenommen Wasserstoff hat,
gegebenenfalls in Gegenwart von Verdünnungsmitteln umsetzt,
oder

e) für den Fall, daß x für 1 steht und A die angegebene Bedeutung hat, 4-Halogen-2-halogencarbonylamino-5-nitrothiazol-Derivate der Formel (VIII)

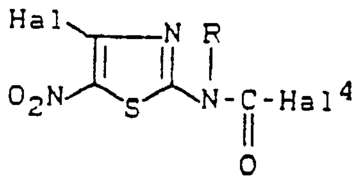

(VIII)

in welcher
$Hal^4$ für Halogen steht,
Hal die oben angegebene Bedeutung hat und
Hal und $Hal^4$ gleich oder verschieden sein können und
R die oben angegebene Bedeutung ausgenommen Wasserstoff hat,
mit Nucleophilen der Formel (IX)
$H-(A)_x-R^1$ (IX)
in welcher
x für 1 steht und
$R^1$ und A die oben angegebene Bedeutung haben,
oder deren Metallsalze gegebenenfalls in Gegenwart von Säurebindemitteln sowie in Gegenwart von Verdünnungsmitteln umsetzt.

Schließlich wurde gefunden, daß die neuen 2-Acylamino-4-halogen-5-nitrothiazol-Derivate der Formel (I)

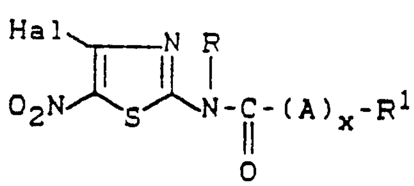

(I)

in welcher
Hal für Halogen steht,
x für eine ganze Zahl 0 oder 1 steht,
A für O, S oder $N-R^2$ steht, worin
$R^2$ für Wasserstoff, Alkyl, Alkenyl, Halogenalkyl, Cyanalkyl, Alkoxycarbonylalkyl oder für jeweils gegebenenfalls ein- bis mehrfach, gleich oder verschieden substituiertes Cycloalkyl, Aryl oder Aralkyl steht,
R für Wasserstoff, Alkyl, Alkenyl oder Alkinyl steht, wobei die vorgenannten Reste jeweils gegebenenfalls ein- bis mehrfach, gleich oder verschieden durch Halogen, Alkoxy, Aryloxy, Alkylmercapto, Arylmercapto und Cyano substituiert sein können, für gegebenenfalls ein- bis mehrfach, gleich oder verschieden durch Alkyl substituiertes Cycloalkyl steht, wobei zusätzlich zu der Alkylsubstitution ein Ring ankondensiert sein kann, für gegebenenfalls ein-bis mehrfach, gleich oder verschieden durch Halogen, Alkyl, Halogenalkyl, Nitro, Alkoxy, Alkylmercapto und Cyan im Arylteil substituiertes Aralkyl steht oder für gegebenenfalls ein- bis mehrfach, gleich oder verschieden durch Halogen, Alkyl, Alkenyl, Alkinyl, Halogenalkyl, Nitro, Alkoxy, Alkylmercapto, Dialkylamino, Carbalkoxy, Carbamoyl, N-Alkylcarbamoyl, N,N-Dialkylcarbamoyl, Halogenalkyloxy, Halogenalkylmercapto, Alkylsulfonylamino, Alkylsulfonyl, Aryl, Aryloxy, Arylmercapto, Acyloxy, Acyl, Sulfamoyl, N-Alkylsulfamoyl, N-Acyl-N-alkylsulfamoyl, N,N-Dialkylsulfamoyl, Aralkyloxy, Aralkylmercapto, Acylamino, Acylaminosulfonyl, Acylalkylamino, Cycloalkyl und Cyan substituiertes Aryl steht und
$R^1$ für Wasserstoff, Alkyl, Alkenyl, Alkinyl, Halogenalkyl, Cyanalkyl, Alkoxycarbonylalkyl oder für jeweils gegebenenfalls ein- bis mehrfach, gleich oder verschieden substituiertes Cycloalkyl, Aryl, Heteroaryl, Aralkyl oder Heteroaralkyl steht oder
R und $R^1$ gemeinsam mit der Gruppierung $-N-CO-(A)_x-$, an der sie stehen, einen Ring bilden, der gegebenenfalls durch ein oder mehrere weitere, gleiche oder verschiedene Heteroatome unterbrochen sein kann und der gegebenenfalls ein- bis mehrfach, gleich oder verschieden substituiert sein kann oder
$R^1$ und $R^2$ gemeinsam mit dem Stickstoffatom, an dem sie stehen, einen Ring bilden, der gegebenenfalls durch ein oder mehrere weitere, gleiche oder verschiedene Heteroatome unterbrochen sein kann und der

gegebenenfalls ein- bis mehrfach, gleich oder verschieden substituiert sein kann und gegebenenfalls weitere Ringe ankondensiert sein können,
mit der Ausnahme, daß $R^1$ nicht Wasserstoff ist, wenn A für O oder S steht,
eine starke Wirkung gegen Schädlinge, vor allem gegen Pilze im Pflanzenbereich und gegen Mikroben in technischen Materialien haben.

Im Rahmen der vorliegenden Erfindung haben die Substituenten die angegebenen Bedeutungen, hier seien einige Substituenten speziell aufgezählt.

Halogen kann, überall wo nicht anders angegeben, Fluor, Chlor, Brom und Iod bedeuten.

Alkyl steht hierbei im allgemeinen in R für einen geradkettigen oder verzweigten Kohlenwasserstoffrest mit 1 bis 12 Kohlenstoffatomen. Genannt seien beispielsweise Methyl, Ethyl, Propyl, Isopropyl, Butyl, Isobutyl, t-Butyl, Pentyl, Isopentyl, Hexyl, Isohexyl, Isoheptyl, Octyl und Isooctyl.

Alkenyl bzw. Alkinyl steht im allgemeinen in R für einen geradkettigen oder verzweigten Kohlenwasserstoffrest mit jeweils bis zu 12 Kohlenstoffatomen und einer oder mehreren, bevorzugt mit einer oder zwei Doppelbindungen bzw. Dreifachbindungen. Beispielsweise seien Allyl, Propenyl, Isopropenyl, Butenyl, Isobutenyl, Pentenyl, Isopentenyl, Hexenyl, Isohexenyl, Heptenyl, Isoheptenyl, Octenyl, Isooctenyl, Propinyl, Butinyl, Pentinyl, Isopentinyl, Hexinyl und Octinyl genannt.

Cycloalkyl steht im allgemeinen in R für einen cyclischen Kohlenwasserstoffrest mit 3 bis 8 Kohlenstoffatomen. Bevorzugt ist der Cyclopropyl-, Cyclopentyl- und der Cyclohexylring. Beispielsweise seien Cyclopropyl, Cyclopentyl, Cyclohexyl, Cycloheptyl und Cyclooctyl genannt.

Halogenalkyl steht in R im allgemeinen für geradkettiges oder verzweigtes Niedrigalkyl mit 1 bis 8 Kohlenstoffatomen und einem bis mehreren, bevorzugt mit 1 bis 10 gleichen oder verschiedenen Halogenatomen. Beispielhaft seien genannt: Fluormethyl, Chlormethyl, Brommethyl, Fluorethyl, Chlorethyl, Bromethyl, Fluorpropyl, Chlorpropyl, Brompropyl, Fluorbutyl, Chlorbutyl, Brombutyl, Fluorisopropyl, Chlorisopropyl, Bromisopropyl, Difluormethyl, Trifluormethyl, Dichlormethyl, Trichlormethyl, Difluorethyl, Dichlorethyl, Trifluorethyl, Tetrafluorethyl, Pentafluorethyl, Trichlorethyl, Trifluorpropyl. Ganz besonders bevorzugt sind Trifluormethyl, Difluormethyl, Fluormethyl, Chlormethyl und Trifluorethyl.

Halogenalkenyl bzw. Halogenalkinyl in R steht im allgemeinen für geradkettiges oder verzweigtes Alkenyl bzw. Alkinyl mit bis zu 8 Kohlenstoffatomen und einem bis 10, gleichen oder verschiedenen Halogenatomen und mit einer oder mehreren Doppelbindungen bzw. Dreifachbindungen. Bevorzugt sind Reste mit einer Doppelbindung bzw. Dreifachbindung. Beispielhaft seien genannt: 2,2-Dichlorvinyl, 1,2,2-Trichlorvinyl, Chlorpropinyl u.a.

Aryl kann für einen aromatischen Kohlenwasserstoffrest mit 6 bis 12 Kohlenstoffatomen stehen. Beispielsweise seien Phenyl oder Naphthyl genannt.

Aralkyl kann für einen Rest mit 7 bis 16 Kohlenstoffatomen stehen, wobei ein geradkettiger oder verzweigter Alkylrest mit 1 bis 4 Kohlenstoffatomen durch einen aromatischen Rest mit 6 bis 12 Kohlenstoffatomen substituiert sein kann. Beispielsweise seien Benzyl, Phenylethyl und Phenylpropyl genannt. Bevorzugt sind Benzyl und Phenylethyl.

Die Aryl- bzw. Aralkylreste können gegebenenfalls ein-bis mehrfach, bevorzugt 1- bis 5-fach, gleich oder verschieden substituiert sein.

Alkoxyalkyl bzw. Alkylmercaptoalkyl stehen im allgemeinen für einen über Sauerstoff bzw. Schwefel gebundenen, geradkettigen oder verzweigten Kohlenwasserstoffrest mit 1 bis 4 Kohlenstoffatomen je Alkyl- bzw. Alkoxy- und Alkylthioteil. Beispielsweise seien genannt Methoxymethyl, Methoxyethyl, Methoxypropyl, Methoxybutyl, Ethoxymethyl, Ethoxyethyl, Ethoxypropyl, Ethoxybutyl, Propoxymethyl, Propoxyethyl, Propoxypropyl, Propoxybutyl, Butoxymethyl, Butoxyethyl, Butoxypropyl und Butoxybutyl.

Ferner Methylthiomethyl, Methylthioethyl, Methylthiopropyl, Methylthiobutyl, Ethoxythiomethyl, Ethylthioethyl, Ethylthiopropyl, Ethylthiobutyl, Propylthiomethyl, Propylthioethyl, Propylthiopropyl, Propylthiobutyl, Butylthiomethyl, Butylthioethyl, Butylthiopropyl und Butylthiobutyl.

Generell gilt, daß aliphatische Reste, wie z.B. Alkyl, Alkoxy, Alkoxycarbonyl u.a. stets geradkettig oder verzweigt sein können.

Für Substitution von Ringen, wie Phenyl, Naphthyl, Heterocyclen u.a. gilt, daß sie im allgemeinen 1- bis 5- fach, besonders bevorzugt 1- bis 3-fach, gleich oder verschieden substituiert sein können, wenn es nicht anders vermerkt ist.

Die erfindungsgemäßen 2-Acylamino-4-halogen-5-nitrothiazol-Derivate sind durch die Formel (I) allgemein definiert. Bevorzugt sind Verbindungen der Formel (I), in welcher
Hal für Chlor, Brom oder Iod steht,
x für eine ganze Zahl 0 oder 1 steht,
A für O, S oder $NR^2$ steht, wobei
$R^2$ für Wasserstoff, Alkyl mit 1 bis 4 Kohlenstoffatomen, Alkenyl mit 3 bis 5 Kohlenstoffatomen, Halogenalkyl

mit 1 bis 3 Kohlenstoffatomen und 1 bis 5 gleichen oder verschiedenen Halogenatomen, für Cyanalkyl mit 1 oder Kohlenstoffatomen im Alkylteil, für Alkoxycarbonylalkyl mit jeweils 1 bis 4 Kohlenstoffatomen im Alkoxy- und Alkylteil, für Cycloalkyl mit 3 bis 6 Kohlenstoffatomen, für gegebenenfalls jeweils ein- bis dreifach, gleich oder verschieden durch Alkyl mit 1 bis 4 Kohlenstoffatomen, Fluor, Chlor, Brom oder Alkoxy mit 1 bis 4 Kohlenstoffatomen substituiertes Phenyl oder Phenylalkyl mit 1 bis 3 Kohlenstoffatomen im Alkylteil steht und

R für Wasserstoff, Alkyl mit 1 bis 12 Kohlenstoffatomen, Alkenyl mit bis zu 12 Kohlenstoffatomen, Alkinyl mit bis zu 12 Kohlenstoffatomen, Halogenalkyl, Halogenalkenyl oder Halogenalkinyl mit jeweils bis zu 8 Kohlenstoffatomen und 1 bis 10, gleichen oder verschiedenen Halogenatomen steht, für Alkoxyalkyl, Alkylmercaptoalkyl oder Cyanalkyl mit 1 bis 4 Kohlenstoffatomen je Alkyl-, Alkoxy-bzw. Alkylthioteil steht, für Phenyloxyalkyl oder Phenylmercaptoalkyl mit 1 bis 4 Kohlenstoffatomen im Alkylteil steht, wobei die Phenylreste gegebenenfalls ein- bis dreifach, gleich oder verschieden durch Fluor, Chlor, Brom oder Alkyl mit 1 bis 4 Kohlenstoffatomen substituiert sein können, für gegebenenfalls ein- bis dreifach, gleich oder verschieden durch Alkyl mit 1 bis 4 Kohlenstoffatomen substituiertes Cycloalkyl mit 3 bis 8 Kohlenstoffatomen steht, wobei der Cycloalkylring zusätzlich einen ankondensierten Ring enthalten kann, für gegebenenfalls ein- bis fünffach, gleich oder verschieden durch Halogen, Alkyl mit 1 bis 4 Kohlenstoffatomen, Halogenalkyl mit 1 bis 4 Kohlenstoffatomen und 1 bis 8 gleichen oder verschiedenen Halogenatomen, Nitro, Cyano, Alkoxy mit 1 bis 4 Kohlenstoffatomen oder Alkylmercapto mit 1 bis 4 Kohlenstoffatomen substituiertes Phenylalkyl mit 1 bis 4 Kohlenstoffatomen im Alkylteil steht, wobei der Alkylteil gegebenenfalls einen weiteren Phenylrest als Substituenten enthält, der auch gegebenenfalls, wie vorher angegeben, substituiert sein kann, oder für gegebenenfalls ein- bis fünffach, gleich oder verschieden durch Halogen, Nitro, Alkenyl oder Alkinyl mit jeweils bis zu 5 Kohlenstoffatomen, Alkyl, Alkoxy, Alkylmercapto, Carbal koxy, Alkylsulfonylamino, Alkylsulfonyl, Sulfamoyl, N-Alkylsulfamoyl, N,N-Dialkylsulfamoyl, Dialkylamino, Carbamoyl, N-Alkylcarbamoyl, N,N-Dialkylcarbamoyl mit jeweils 1 bis 4 Kohlenstoffatomen je Alkylrest, Halogenalkyl, Halogenalkoxy, Halogenalkylmercapto mit jeweils bis 4 Kohlenstoffatomen und 1 bis 8 gleichen oder verschiedenen Halogenatomen je aufgezähltem Rest, Phenyl, Phenoxy, Phenylmercapto, Acyloxy mit 1 bis 3 Kohlenstoffatomen, Acyl mit 1 bis 3 Kohlenstoffatomen, Phenylalkyloxy mit 1 bis 3 Kohlenstoffatomen im Alkylteil, Phenylalkylmercapto mit 1 bis 3 Kohlenstoffatomen, Acylamino mit 1 bis 3 Kohlenstoffatomen, Acylalkylamino, N-Acyl-alkylsulfamoyl oder Acylaminosulfonyl mit jeweils 1 bis 3 Kohlenstoffatomen je Acyl- und Alkylrest, Cycloalkyl mit 4 bis 6 Kohlenstoffatomen und Cyan substituiertes Phenyl oder für Naphthyl steht,

$R^1$ für Wasserstoff, Alkyl mit 1 bis 6 Kohlenstoffatomen, Alkenyl oder Alkinyl mit jeweils 3 bis 5 Kohlenstoffatomen, Halogenalkyl mit 1 bis 5 Kohlenstoffatomen und 1 bis 8 gleichen oder verschiedenen Halogenatomen, Cyanalkyl mit 1 oder 2 Kohlenstoffatomen im Alkylteil, Alkoxycarbonylalkyl mit jeweils 1 bis 4 Kohlenstoffatomen im Alkoxy- und Alkylteil, für gegebenenfalls ein- bis dreifach durch Alkyl mit 1 bis 3 Kohlenstoffatomen substituiertes Cycloalkyl mit 3 bis 6 Kohlenstoffatomen, für gegebenenfalls jeweils ein- bis dreifach, gleich oder verschieden durch Alkyl mit 1 bis 4 Kohlenstoffato men, Fluor, Chlor, Brom, Alkoxy, Halogenalkyl oder Halogenalkoxy mit je 1 bis 4 Kohlenstoffatomen und gegebenenfalls 1 bis 5 gleichen oder verschiedenen Halogenatomen substituiertes Phenyl oder Phenylalkyl mit 1 bis 3 Kohlenstoffatomen im Alkylteil oder für Heterocyclyl oder Heterocyclylalkyl mit 1 bis 4 Kohlenstoffatomen im Alkylteil und 5 bis 7 Ringgliedern im Heterocyclylteil, der ein bis drei, gleiche oder verschiedene Heteroatome, z.B. Sauerstoff, Schwefel oder Stickstoff, enthalten kann und denen weitere Ringe ankondensiert sein können, steht, wobei die Ringe jeweils gegebenenfalls gleich oder verschieden, ein- bis dreifach substituiert sein können durch Halogen, Alkyl mit 1 bis 4 Kohlenstoffatomen und Alkoxy mit 1 bis 3 Kohlenstoffatomen, oder

R und $R^1$ gemeinsam mit der Gruppierung $-N-CO-(A)_x-$, an der sie stehen, einen Ring mit 5 bis 7 Ringgliedern bilden, der gegebenenfalls ein oder zwei weitere Stickstoff- und/oder Sauerstoffatome enthalten kann und gegebenenfalls ein- bis dreifach durch Alkyl mit 1 bis 4 Kohlenstoffatomen substituiert sein kann, oder

$R^1$ und $R^2$ gemeinsam mit dem Stickstoffatom, an dem sie stehen, einen Ring mit 5 bis 7 Ringgliedern bilden, der gegebenenfalls ein oder zwei weitere Stickstoff- und/oder Sauerstoffatome enthalten kann und gegebenenfalls ein- bis dreifach durch Alkyl mit 1 bis 4 Kohlenstoffatomen substituiert sein kann und weitere Ringe ankondensiert sein können, mit der Ausnahme, daß $R^1$ nicht Wasserstoff ist, wenn A für O oder S steht und ausgenommen die Verbindung 2-Acetylamino-4-iodo-5-nitro-thiazol.

Besonders bevorzugt sind Verbindungen der Formel (I), in welcher

Hal für Chlor steht,

x für 0 steht,

R für Wasserstoff, geradkettiges oder verzweigtes Alkyl mit 1 bis 6 Kohlenstoffatomen, geradkettiges oder verzweigtes Alkenyl mit bis zu 6 Kohlenstoffatomen, Halogenalkyl mit 1 bis 4 Kohlenstoffatomen und 1 bis 6

gleichen oder verschiedenen Halogenatomen, Alkoxyalkyl mit jeweils 1 bis 3 Kohlenstoffatomen je Alkoxy- bzw. Alkylteil, ferner für Cyclohexyl, Naphthyl, gegebenenfalls ein- bis dreifach, gleich oder verschieden durch Alkyl mit 1 bis 3 Kohlenstoffatomen und Halogen substituiertes Phenylalkyl mit 1 bis 4 Kohlenstoffatomen im geradkettigen oder verzweigten Alkylteil, oder für gegebenenfalls ein- bis fünffach, gleich oder verschieden durch Alkyl mit 1 bis 3 Kohlenstoffatomen oder gegebenenfalls ein- bis dreifach, gleich oder verschieden durch Halogen, Nitro, Cyclohexyl, Alkyl mit 1 bis 4 Kohlenstoffatomen, Alkoxy mit 1 bis 4 Kohlenstoffatomen, Alkylsulfonyl mit 1 bis 4 Kohlenstoffatomen, Dialkylamino mit 1 bis 4 Kohlenstoffatomen je Alkylteil, Carbamoyl, N-Alkyl- oder N,N-Dialkyl-carbamoyl mit 1 bis 4 Kohlenstoffatomen je Alkylteil, Halogenalkoxy oder Halogenalkylmercapto mit jeweils 1 bis 4 Kohlenstoffatomen und 1 bis 5 gleichen oder verschiedenen Halogenatomen, Acyl oder Acylamino mit jeweils 1 bis 3 Kohlenstoffatomen, Acyloxy mit 1 bis 3 Kohlenstoffatomen, N-Acyl-alkylsulfamoyl oder Acylaminosulfonyl mit jeweils 1 bis 3 Kohlenstoffatomen je Acyl- und Alkylteil substituiertes Phenyl steht und

R' für Alkyl mit 1 bis 6 Kohlenstoffatomen, Halogenalkyl mit 1 bis 3 Kohlenstoffatomen und 1 bis 5 gleichen oder verschiedenen Halogenatomen, für gegebenenfalls 1- bis 3-fach durch Methyl, Ethyl oder Propyl substituiertes Cyclohexyl, für gegebenenfalls jeweils ein- bis dreifach, gleich oder verschieden durch Alkyl mit 1 bis 3 Kohlenstoffatomen, Fluor, Chlor, Brom, Alkoxy, Halogenalkyl oder Halogenalkoxy mit je 1 bis 3 Kohlenstoffatomen und gegebenenfalls 1 bis 5 gleichen oder verschiedenen Halogenatomen substituiertes Phenyl oder für einen gegebenenfalls ein- bis dreifach, gleich oder verschieden durch Chlor, Methyl, Ethyl, n- oder iso-Propyl substituierten 5- oder 6-gliedrigen Ring mit 1 bis 3 gleichen oder verschiedenen Heteroatomen, wie Stickstoff, Schwefel oder Sauerstoff, steht, wie z.B. Oxazol, Isoxazol, Thiazol, Imidazol, Triazol, Pyrazol, Pyridin, Morpholin, Pyrazin.

Weiterhin sind besonders bevorzugt Verbindungen der Formel (I), in welcher

R für Wasserstoff, geradkettiges oder verzweigtes Alkyl mit 1 bis 6 Kohlenstoffatomen, geradkettiges oder verzweigtes Alkenyl mit bis zu 6 Kohlenstoffatomen, Halogenalkyl mit 1 bis 4 Kohlenstoffatomen und 1 bis 6 gleichen oder verschiedenen Halogenatomen, Alkoxyalkyl mit jeweils 1 bis 3 Kohlenstoffatomen je Alkoxy- bzw. Alkylteil, ferner für Cyclohexyl, Naphthyl, gegebenenfalls ein- bis dreifach, gleich oder verschieden durch Alkyl mit 1 bis 3 Kohlenstoffatomen und Halogen substituiertes Phenylalkyl mit 1 bis 4 Kohlenstoffatomen im geradkettigen oder verzweigten Alkylteil, oder für gegebenenfalls ein- bis fünffach, gleich oder verschieden durch Alkyl mit 1 bis 3 Kohlenstoffatomen oder gegebenenfalls ein- bis dreifach, gleich oder verschieden durch Halogen, Nitro, Cyclohexyl, Alkyl mit 1 bis 4 Kohlenstoffatomen, Alkoxy mit 1 bis 4 Kohlenstoffatomen, Alkylsulfonyl mit 1 bis 4 Kohlenstoffatomen, Dialkylamino mit 1 bis 4 Kohlenstoffatomen je Alkylteil, Carbamoyl, N-Alkyl- oder N,N-Dialkyl-carbamoyl mit 1 bis 4 Kohlenstoffatomen je Alkylteil, Halogenalkoxy oder Halogenalkylmercapto mit jeweils 1 bis 4 Kohlenstoffatomen und 1 bis 5 gleichen oder verschiedenen Halogenatomen, Acyl oder Acylamino mit jeweils 1 bis 3 Kohlenstoffatomen, Acyloxy mit 1 bis 3 Kohlenstoffatomen, N-Acyl-alkylsulfamoyl oder Acylaminosulfonyl mit jeweils 1 bis 3 Kohlenstoffatomen je Acyl- und Alkylteil substituiertes Phenyl steht und

x für 1 steht,

A für O oder S steht und

R' für Alkyl mit bis 4 Kohlenstoffatomen, für gegebenenfalls ein- bis dreifach, gleich oder verschieden durch Halogen, Alkoxy, Alkyl, Halogenalkyl und Halogenalkoxy mit jeweils 1 bis 3 Kohlenstoffatomen und gegebenenfalls mit 1 bis 5 gleichen oder verschiedenen Halogenatomen substituiertes Phenyl oder für einen 5- oder 6-gliedrigen Ring mit 1 oder 2 gleichen oder verschiedenen Heteroatomen, wie Stickstoff und Sauerstoff, steht

oder

R und $R^1$ mit der Gruppierung $-N-CO-(A)_x-$, an der sie stehen, einen 5- oder 6-gliedrigen Ring bilden, der gegebenenfalls 1- bis 3-fach, gleich oder verschieden substituiert sein kann durch Halogen und Alkyl mit 1 bis 4 Kohlenstoffatomen, wie z.B. 2-Keto-pyrrolidin und 2-Keto-piperidin.

Weiterhin sind bevorzugt Verbindungen der Formel (I) in der

R für Wasserstoff, geradkettiges oder verzweigtes Alkyl mit 1 bis 6 Kohlenstoffatomen, geradkettiges oder verzweigtes Alkenyl mit bis zu 6 Kohlenstoffatomen, Halogenalkyl mit 1 bis 4 Kohlenstoffatomen und 1 bis 6 gleichen oder verschiedenen Halogenatomen, Alkoxyalkyl mit jeweils 1 bis 3 Kohlen stoffatomen je Alkoxy- bzw. Alkylteil, ferner für Cyclohexyl, Naphthyl, gegebenenfalls ein- bis dreifach, gleich oder verschieden durch Alkyl mit 1 bis 3 Kohlenstoffatomen und Halogen substituiertes Phenylalkyl mit 1 bis 4 Kohlenstoffatomen im geradkettigen oder verzweigten Alkylteil, oder für gegebenenfalls ein- bis fünffach, gleich oder verschieden durch Alkyl mit 1 bis 3 Kohlenstoffatomen oder gegebenenfalls ein- bis dreifach, gleich oder verschieden durch Halogen, Nitro, Cyclohexyl, Alkyl mit 1 bis 4 Kohlenstoffatomen, Alkoxy mit 1 bis 4 Kohlenstoffatomen, Alkylsulfonyl mit 1 bis 4 Kohlenstoffatomen, Dialkylamino mit 1 bis 4 Kohlenstoffatomen je Alkylteil, Carbamoyl, N-Alkyl- oder N,N-Dialkyl-carbamoyl mit 1 bis 4 Kohlenstoffatomen je Alkylteil,

Halogenalkoxy oder Halogenalkylmercapto mit jeweils 1 bis 4 Kohlenstoffatomen und 1 bis 5 gleichen oder verschiedenen Halogenatomen, Acyl oder Acylamino mit jeweils 1 bis 3 Kohlenstoffatomen, Acyloxy mit 1 bis 3 Kohlenstoffatomen, N-Acyl-alkylsulfamoyl oder Acylaminosulfonyl mit jeweils 1 bis 3 Kohlenstoffatomen je Acyl- und Alkylteil substituiertes Phenyl steht und

x für 1 steht,

A für $NR^2$ steht,

$R^1$ für Wasserstoff, Alkyl mit 1 bis 6 Kohlenstoffatomen, Halogenalkyl mit 1 bis 3 Kohlenstoffatomen und 1 bis 5 gleichen oder verschiedenen Halogenatomen, für gegebenenfalls 1- bis 3-fach durch Methyl, Ethyl oder Propyl substituiertes Cyclohexyl, für gegebenenfalls jeweils ein- bis dreifach, gleich oder verschieden durch Alkyl mit 1 bis 3 Kohlenstoffatomen, Fluor, Chlor, Brom, Alkoxy, Halogenalkyl oder Halogenalkoxy mit je 1 bis 3 Kohlenstoffatomen und gegebenenfalls 1 bis 5, gleichen oder verschiedenen Halogenatomen substituiertes Phenyl oder für einen gegebenenfalls ein- bis dreifach, gleich oder verschieden durch Chlor, Methyl, Ethyl, n- oder iso-Propyl substituierten 5- oder 6-gliedrigen Ring mit 1 bis 3 gleichen oder verschiedenen Heteroatomen, wie Stickstoff, Schwefel oder Sauerstoff, steht,

$R^2$ für Wasserstoff, Alkyl mit 1 bis 4 Kohlenstoffatomen, Alkenyl mit 3 bis 5 Kohlenstoffatomen, Halogenalkyl mit 1 bis 3 Kohlenstoffatomen und 1 bis 5 gleichen oder verschiedenen Halogenatomen, für Cyanalkyl mit 1 oder 2 Kohlenstoffatomen im Alkylteil, für Cycloalkyl mit 3 bis 6 Kohlenstoffatomen, für gegebenenfalls jeweils ein- bis dreifach, gleich oder verschieden durch Alkyl mit 1 bis 4 Kohlenstoffatomen, Fluor, Chlor, Brom oder Alkoxy mit 1 bis 4 Kohlenstoffatomen substituiertes Phenyl oder Phenylalkyl mit 1 bis 3 Kohlenstoffatomen im Alkylteil steht und

$R^1$ und $R^2$ gemeinsam mit dem Stickstoffatom, an dem sie stehen, einen Ring mit 5 bis 7 Ringgliedern bilden, der gegebenenfalls ein oder zwei weitere Stickstoff- und/oder Sauerstoffatome enthalten kann und gegebenenfalls ein- bis dreifach durch Alkyl mit 1 bis 4 Kohlenstoffatomen substituiert sein kann und weitere Ringe ankondensiert sein können.

Verwendet man bei dem erfindungsgemäßen Verfahren (a) z.B. 2,4-Dichlor-5-nitrothiazol und N,N′-Dimethylharnstoff als Ausgangsstoffe, so kann der Verlauf der Umsetzung durch das folgende Formelschema wiedergegeben werden:

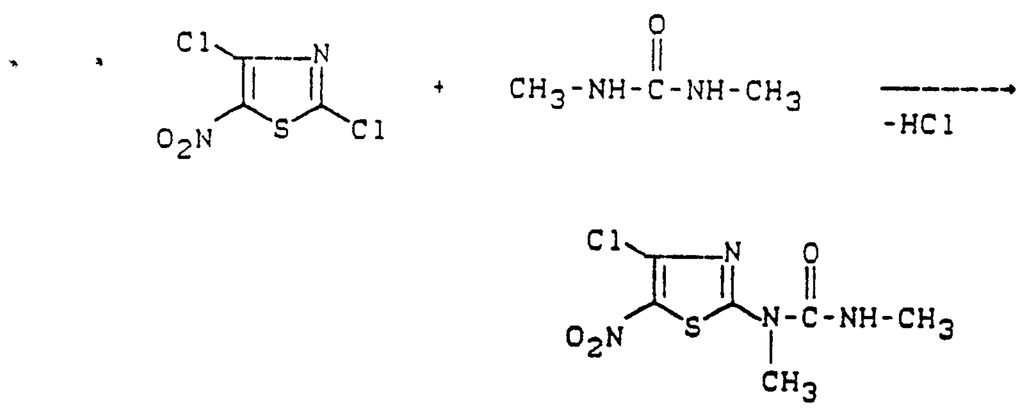


Verwendet man z.B. 4-Chlor-2-ethylamino-5-nitrothiazol und Chloracetylchlorid als Ausgangsstoffe, so kann der Verlauf des erfindungsgemäßen Verfahrens (b) durch das folgende Formelschema wiedergegeben werden:

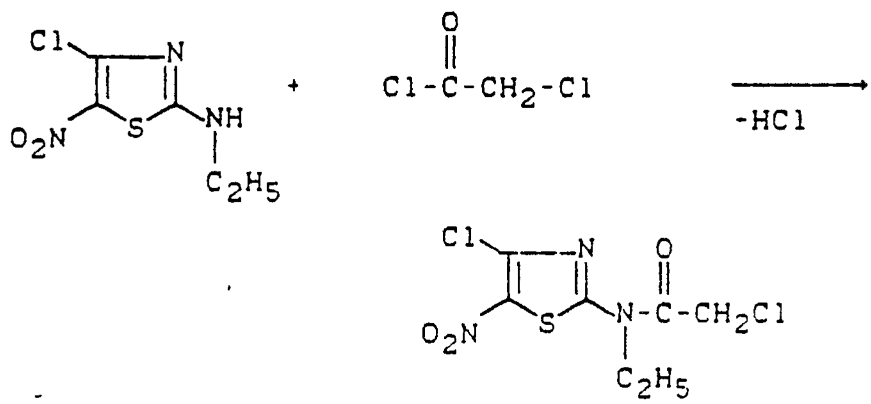


Verwendet man z.B. 2-Anilino-4-chlor-5-nitrothiazol und Acetanhydrid als Ausgangsstoffe, so kann der Verlauf des erfindungsgemäßen Verfahrens (c) durch das folgende Formelschema wiedergegeben werden:

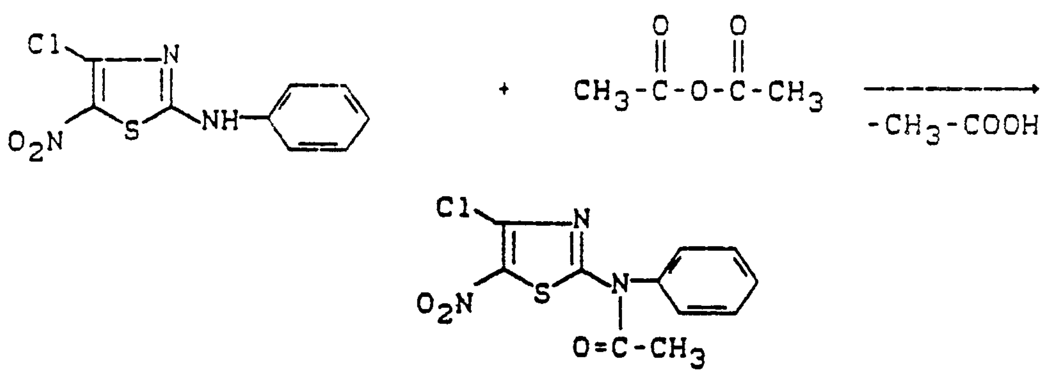


Verwendet man z.B. 4-Chlor-2-methylamino-5-nitrothiazol und Phenylisocyanat als Ausgangsstoffe, so kann der Verlauf des erfindungsgemäßen Verfahrens (d) durch das folgende Formelschema wiedergegeben werden:

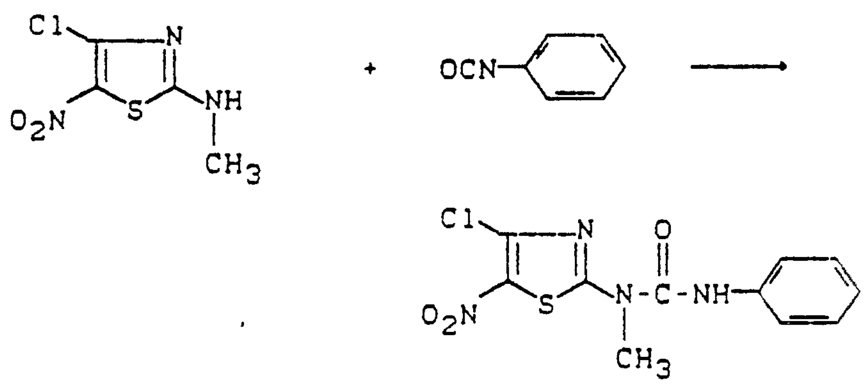

Verwendet man z.B. 4-Chlor-2-(N-chlorcarbonyl-N-methyl)amino-5-nitrothiazol und Methanol als Ausgangsstoffe, so kann der Verlauf des erfindungsgemäßen Verfahrens (e) durch das folgende Formelschema wiedergegeben werden:

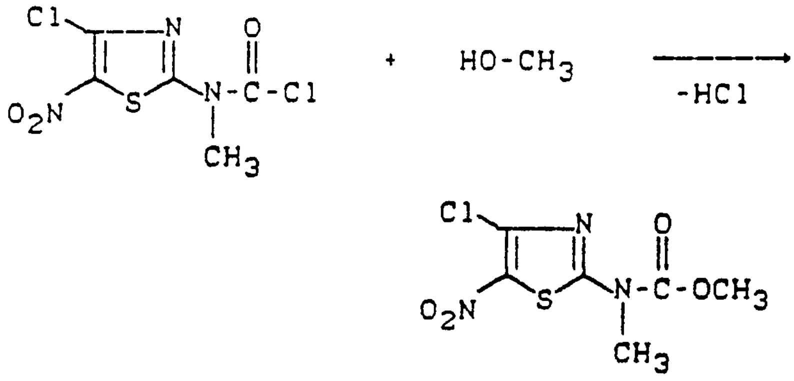


Die bei dem erfindungsgemäßen Verfahren (a) als Ausgangsprodukte benötigten 2,4-Dihalogen-5-nitrothiazole der Formel (II) sind teilweise bekannt, z. B. für Verbindungen der Formel (II), in welcher
$Hal = Hal^1$ = Brom steht: Chem. Abstr. 61: 3087 f und für
$Hal = Hal^1$ = Chlor steht, vergl. DE-OS 3 518 520.

Die beiden genannten Verbindungen sind z. B. durch Nitrierung der entsprechenden 2,4-Dihalogenthiazole zugänglich.

Verbindungen der Formel (II A)

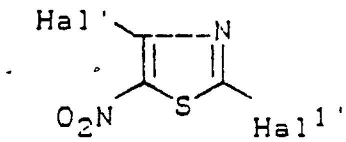

(II A)

in welcher
$Hal'$ und $Hal^1$ unabhängig voneinander für Chlor, Iod oder Fluor stehen, mit der Maßgabe, daß nicht beide Hals gleichzeitig Chlor sein dürfen,
sind Gegenstand einer nicht vorveröffentlichten Deutschen Anmeldung P 38 24 520.5. Bei entsprechenden Anwendungskonzentrationen zeigen auch diese Stoffe eine mikrobizide Wirkung.

So sind Verbindungen der Formel (II A), in der $Hal' = Hal^1$ = Iod oder
$Hal'$ = Chlor und $Hal^1$ = Iod oder
$Hal'$ = Iod und $Hal^1$ = Chlor oder
$Hal'$ = Chlor und $Hal^1$ = Fluor
bedeuten, z. B. neu. So werden die Iodverbindungen durch Umsetzung von 2,4-Dichlor-5-nitrothiazol mit Metalliodiden, insbesondere Natriumiodid, in niederen aliphatischen Ketonen, insbesondere Aceton, als Lösungsmittel entsprechend den folgenden Formelschemata gewonnen:

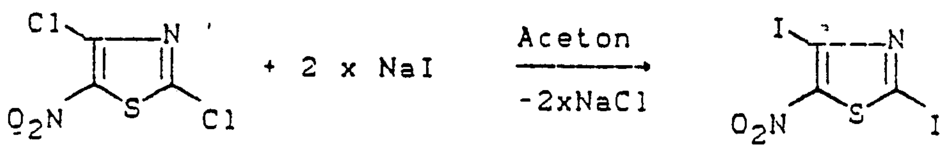


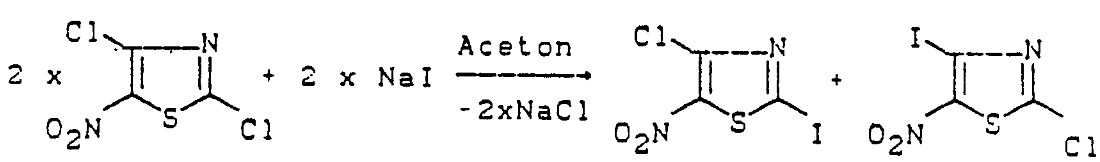

Die beiden isomeren Chlor-iod-nitrothiazole entstehen nebeneinander und können durch fraktionierte Kristallisation und/oder durch chromatographische Methoden voneinander getrennt werden. Die Reaktionstemperatur liegt im allgemeinen zwischen 0 °C und 130 °C, vorzugsweise zwischen 10 °C und 90 °C.

Verbindungen der Formel (II A), in der mindestens eines der Halogene Fluor ist, z. B. Hal[1] = Fluor und Hal′ = Chlor, also das 4-Chlor-2-fluor-5-nitrothiazol können hergestellt werden, indem man 2,4-Dichlor-5-nitrothiazol mit Metallfluoriden, insbesondere Natriumfluorid oder Kaliumfluorid, in niederen aliphatischen Nitrilen, insbesondere in Acetonitril oder Propionitril als Lösungsmittel in Gegenwart katalytischer Mengen von Kronenethern, insbesondere von [18] Krone-6 umsetzt. Die Reaktionstemperatur beträgt 0 °C bis 50 °C, vorzugsweise 10 °C bis 30 °C. Man arbeitet mit 2 - 15 Mol Metallfluorid, vorzugsweise 3 - 12 Mol Metallfluorid pro Mol 2,4-Dichlor-5-nitrothiazol.

Die bei dem erfindungsgemäßen Verfahren (a) weiterhin als Ausgangsprodukte benötigten Nucleophile der Formel (III) sind grundsätzlich bekannt. Falls spezielle Verbindungen noch nicht beschrieben sind, lassen sie sich nach bekannten Verfahren herstellen.

Als Lösungsmittel kommen bei der Durchführung des erfindungsgemäßen Verfahrens (a) insbesondere aprotische, dipolare Lösungsmittel in Frage wie Dimethylformamid, Dimethylacetamid, N-Methyl-2-pyrrolidinon, N-Methylcaprolactam, Tetramethylharnstoff, N,N′-Dimethyl-1,3-imidazolidin-2-on, Hexamethylphosphorsäuretrisamid, Dimethylsulfoxid, Dimethylsulfon sowie Tetramethylensulfon ( = Sulfolan).

Die Reaktionstemperatur kann bei dem erfindungsgemäßen Verfahren (a) in weiten Bereichen variiert werden. Im allgemeinen arbeitet man zwischen -20 °C und +150 °C, vorzugsweise bei 0 °C bis 100 °C.

Die Umsetzungen werden in der Regel bei Normaldruck ausgeführt, jedoch kann auch in geschlossenen Gefäßen ge arbeitet werden, in denen sich dann in Abhängigkeit von der Art des Lösungsmittels und von der Temperatur ein entsprechend höherer Druck einstellt.

Zur Bindung des bei dem erfindungsgemäßen Verfahren (a) gemäß Formelschema freiwerdenden Halogenwasserstoffs ist im allgemeinen ein säurebindendes Mittel erforderlich, man kann aber auch ohne arbeiten. Hierfür sind z.B. verwendbar: Alkalihydroxide, -carbonate oder -hydrogencarbonate oder auch Alkalihydride, vgl. dazu auch den folgenden Absatz.

Es können auch direkt Metallsalze, vorzugsweise Alkalisalze des betreffenden Nucleophils der Formel (III) zur Reaktion gebracht werden. Die Salze können entweder als solche eingesetzt werden oder, beispielsweise durch Zugabe von Alkalihydriden, vorzugsweise Natriumhydrid, in situ erzeugt werden.

Wenn ohne säurebindendes Mittel gearbeitet wird, sind die aprotischen, dipolaren Lösungsmittel Dimethylformamid und N-Methyl-2-pyrrolidinon besonders bevorzugt. Arbeitet man aber z.B. mit Natriumhydrid, sind darüber hinaus cyclische Ether wie Dioxan und Tetrahydrofuran als Lösungsmittel besonders bevorzugt.

Die Reaktionspartner werden im allgemeinen im stöchiometrischen äquimolaren Verhältnis miteinander zur Reaktion gebracht.

Da die 2,4-Dihalogen-5-nitrothiazole der Formel (II) in 4-Stellung ein zweites - wenn auch abgestuft deutlich weniger reaktives - Halogenatom tragen, ist es im allgemeinen zweckmäßig, keinen Überschuß an Nucleophil der Formel (III) einzusetzen, um die Bildung hier unerwünschter Disubstitutionsprodukte auszuschließen. Deshalb kann es in bestimmten Fällen vorteilhaft sein, einen Überschuß an 2,4-Dihalogen-5-nitrothiazol der Formel (II) einzusetzen, der im allgemeinen 20 bis zu 100 Mol-% betragen kann.

Bei der Durchführung des erfindungsgemäßen Verfahrens (a) wird bevorzugt das 2,4-Dihalogen-5-nitrothiazol der Formel (II) in einem der genannten Lösungsmittel vorgelegt. Arbeitet man ohne säurebindendes Mittel, so wird anschließend bei Raumtemperatur das Nucleophil der Formel (III) zugegeben und auf die gewünschte End-Reaktionstemperatur erhitzt. Arbeitet man in Gegenwart eines säurebindenden Mittels - besonders bevorzugt Natriumhydrid -, so kann man in verschiedener Weise verfahren. Entweder man erzeugt in einem getrennten Reaktionsgefäß zunächst in situ - bevorzugt in einem cyclischen Ether wie Tetrahydrofuran - das Alkalisalz des Nucleophils der Formel (III) und fügt dieses anschließend bei Raumtemperatur oder darunter, bevorzugt bis 0 °C, zur Lösung des 2,4-Dihalogen-5-nitrothiazols der Formel (II) oder man kann zur Lösung des 2,4-Dihalogen-5-nitrothiazols der Formel (II) zuerst das Nucleophil der Formel (III) und danach - zweckmäßig portionsweise - das säurebindende Mittel - besonders bevorzugt Natriumhydrid - geben oder zuerst das säurebindende Mittel und danach - wiederum zweckmäßig portionsweise - das Nucleophil der Formel (III) hinzufügen.

Anschließend wird so lange bei der gewünschten Reaktionstemperatur gerührt, bis die Umsetzung beendet ist.

Die Isolierung der Reaktionsprodukte erfolgt nach üblichen Methoden. Im einfachsten Fall wird das Reaktionsgemisch gegebenenfalls nach teilweisem oder völligem Abdestillieren des Lösungsmittels im Vakuum unterhalb der Reaktionstemperatur in überschüssigem Eiswasser, etwa der 5- bis 10-fachen Volumenmenge des eingesetzten Lösungsmittels, verrührt, abfiltriert, mit Wasser gewaschen und getrocknet. Wenn mit einem Überschuß 2,4-Dihalogen-5-nitrothiazol gearbeitet wurde, kann dies aus dem gut getrockneten Rohprodukt durch Verrühren bei Raumtemperatur mit einem Lösungsmittel, das praktisch nur das 2,4-Dihalogen-5-nitrothiazol der Formel (II) löst, herausgelöst und gegebenenfalls für Folgeansätze wiederverwendet werden. Im Falle des 2,4-Dichlor-5-nitrothiazols ist z.B. Petrolether besonders gut geeignet. Falls das Reaktionsprodukt, das noch überschüssiges 2,4-Dihalogen-5-nitrothiazol der Formel (II) enthält, selbst in Petrolether bei Raumtemperatur gut löslich ist, wird das 2,4-Dihalogen-5-nitrothiazol der Formel (II) z. B. im Vakuum bei etwa 0,1 mbar fraktioniert von dem meist deutlich schwerer flüchtigen Reaktionsprodukt absublimiert bzw. abdestilliert. Im Falle des 2,4-Dichlor-5-nitrothiazols ist dies z. B. bei einer Temperatur von etwa 70 °C und 0,1 mbar möglich. Eine weitere Reinigung der Reaktionsprodukte kann z.B. durch Umkristallisie ren, durch Sublimation oder auf chromatographischem Wege erfolgen.

Falls ein Reaktionsprodukt beim Verrühren in überschüssigem Eiswasser als Öl anfällt, wird es durch mehrmaliges Ausschütteln mit einem der gebräuchlichen organischen, nicht mit Wasser mischbaren Lösungsmitteln wie z.B. Methylenchlorid isoliert, indem man die organische Phase abtrennt, gegebenenfalls mit Wasser wäscht, z.B. im Falle von Dimethylformamid oder N-Methyl-2-pyrrolidinon als Reaktionsmedium, dann trocknet und im Vakuum einengt.

Die bei dem erfindungsgemäßen Verfahren (b) als Ausgangsstoffe benötigten 2-Amino-4-halogen-5-nitrothiazol-Derivate der Formel (IV) sind Gegenstand einer eigenen, nicht vorveröffentlichten Deutschen Anmeldung P 38 24 520.5 vom 20.07.1988.

Man erhält sie, indem man 2,4-Dihalogen-5-nitrothiazole der Formel (II) mit Nucleophilen der Formel (X)

$H_2N-R$ (X)

in welcher

R die oben angegebene Bedeutung hat,

gegebenenfalls in Gegenwart von Säurebindemitteln sowie in Gegenwart von Verdünnungsmitteln umsetzt. Präparative Einzelheiten dieser Reaktionen sind den Herstellungsbeispielen zu entnehmen.

Die bei dem erfindungsgemäßen Verfahren (b) weiterhin als Ausgangsstoffe benötigten Halogencarbonyl-Verbindungen der Formel (V) sind grundsätzlich bekannt. Falls spezielle Verbindungen noch nicht beschrieben sind, lassen sie sich nach bekannten Verfahren herstellen.

Das als Ausgangsprodukt für Verbindung Nr. 19 verwendete 4-Chlorcarbonyl-2,5-dichlorthiazol ist Gegenstand der nicht vorveröffentlichten Deutschen Patentanmeldung P 38 21 598.5 vom 27.06.1988. Ausgehend vom literaturbekannten 2-Chlor-4-methylthiazol (J. Chem. Soc. 1919, 1071-1090) wird es nach folgendem Formelschema hergestellt:

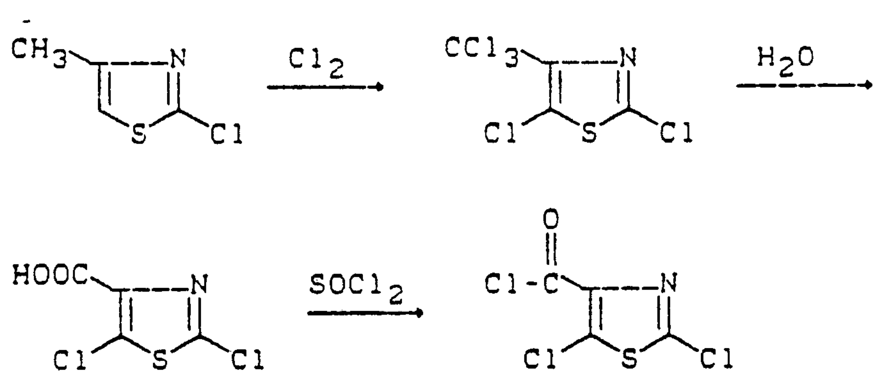


Präparative Einzelheiten dieser Reaktionen sind den Herstellungsbeispielen zu entnehmen.

Als Lösungsmittel kommen bei der Durchführung des erfindungsgemäßen Verfahrens (b) in Frage: Aliphatische oder aromatische Nitrile, z. B. Acetonitril, Propionitril, Benzonitril, insbesondere Acetonitril, Ether, insbesondere cyclische Ether wie Dioxan, Tetrahydrofuran, insbesondere Dioxan.

Die Reaktionstemperatur kann bei dem erfindungsgemäßen Verfahren (b) in weiten Bereichen variiert werden. Im allgemeinen arbeitet man zwischen -20 °C und +150 °C, vorzugsweise bei 0 °C bis 100 °C.

Die Umsetzungen werden in der Regel bei Normaldruck ausgeführt, jedoch kann auch in geschlossenen Gefäßen gearbeitet werden, in denen sich dann in Abhängigkeit von der Art des Lösungsmittels und von der Temperatur ein entsprechend höherer Druck einstellt.

Zur Bindung des. bei dem erfindungsgemäßen Verfahren (b) gemäß Formelschema freiwerdenden

Halogenwasserstoffs ist nicht in allen Fällen ein säurebindendes Mittel erforderlich. Arbeitet man ohne säurebindendes Mittel, so wählt man zweckmäßig als Reaktionstemperatur die Rückflußtemperatur des jeweiligen Lösungsmittels. Falls säurebindende Mittel verwendet werden, kommen hierfür in Frage: Alkalihydroxide, -carbonate, -hydrogencarbonate oder Alkalihydride, z.B. Natriumhydrid. In diesen Fällen ist als Reaktionstemperatur Raumtemperatur besonders geeignet.

Die Reaktionspartner werden im allgemeinen im stöchiometrisch äquimolaren Verhältnis miteinander zur Reaktion gebracht, jedoch kann man auch einen Überschuß an Halogencarbonyl-Verbindung der Formel (V) einsetzen, der bis zu 100 % betragen kann.

Bei der Durchführung des erfindungsgemäßen Verfahrens (b) wird das 2-Amino-4-halogen-5-nitrothiazol-Derivat der Formel (IV) vorzugsweise in einem der genannten Lösungsmittel gelöst, vorzugsweise die äquimolare bis 1.5-fach molare Menge an Halogencarbonyl-Verbindung der Formel (V) zugefügt, gegebenenfalls die äquimolare bis 1.5-fach molare Menge eines der genannten säurebindenden Mittel zugefügt und so lange bei der gewünschten Reaktionstemperatur gerührt, bis die Umsetzung beendet ist. Der Verlauf der Umsetzung läßt sich leicht dünnschichtchromatographisch verfolgen. Die Reaktionszeiten betragen je nach Reaktionspartner und Reaktionsbedingungen zwischen einer und hundert Stunden.

Die bei dem erfindungsgemäßen Verfahren (c) als Ausgangsstoffe zusätzlich benötigten Carbonsäureanhydride der Formel (VI) sind grundsätzlich bekannt. Falls spezielle Verbindungen noch nicht beschrieben sind, lassen sie sich nach bekannten Verfahren herstellen.

Als Lösungsmittel kommen bei der Durchführung des erfindungsgemäßen Verfahrens (c) in Frage: Aliphatische oder aromatische Nitrile, z. B. Acetonitril, Propionitril, Benzonitril, insbesondere Acetonitril, Ether, insbesondere cyclische Ether wie Dioxan, Tetrahydrofuran, insbesondere Dioxan.

Die Reaktionstemperatur kann bei dem erfindungsgemäßen Verfahren (c) in weiten Bereichen variiert werden. Im allgemeinen arbeitet man zwischen 50 °C und 200 °C, vorzugsweise bei 60 °C bis 150 °C.

Die Umsetzungen werden in der Regel bei Normaldruck ausgeführt, jedoch kann auch in geschlossenen Gefäßen gearbeitet werden, in denen sich dann in Abhängigkeit von der Art des Lösungsmittels und von der Temperatur ein entsprechend höherer Druck einstellt.

Bei dem erfindungsgemäßen Verfahren (c) werden als Ausgangsstoffe bevorzugt aliphatische Carbonsäureanhydride eingesetzt. In diesen Fällen ist es besonders bevorzugt, statt eines der genannten Lösungsmittel überschüssiges aliphatisches Carbonsäureanhydrid zu verwenden. Im allgemeinen werden dann pro Gramm zu acylierender Ausgangsverbindung (IV) 5 bis 20 ml aliphatisches Carbonsäureanhydrid eingesetzt und so lange unter Rückfluß erhitzt, bis die Umsetzung beendet ist. Die Reaktionszeiten betragen je nach Reaktionspartner und Reaktionsbedingungen zwischen einer und hundert Stunden.

Ist R in Formel (IV) ein Aryl-, insbesondere ein Phenylrest, und ist dieser durch Hydroxylgruppen oder Aminogruppen substituiert, so werden diese bei der Umsetzung in überschüssigem aliphatischem Carbonsäureanhydrid, insbesondere in Acetanhydrid, mitacyliert. Präparative Einzelheiten dieser Reaktionen sind den Herstellungsbeispielen zu entnehmen.

Falls mit überschüssigem aliphatischem Carbonsäureanhydrid gearbeitet wird, ist die Aufarbeitung besonders einfach. Man engt im Vakuum, z.B. in einem Rotavapor, zur Trockne ein. Um letzte Reste Carbonsäureanhydrid zu entfernen, wird entweder zwischen Tonplatten bis zur Ge wichtskonstanz getrocknet oder man verrührt in Wasser, filtriert ab und trocknet.

Die bei dem erfindungsgemäßen Verfahren (d) als Ausgangsstoffe benötigten Isocyanate der Formel (VII) sind grundsätzlich bekannt. Falls spezielle Verbindungen noch nicht beschrieben sind, lassen sie sich nach bekannten Verfahren herstellen.

Als Lösungsmittel kommen bei der Durchführung des erfindungsgemäßen Verfahrens (d) in Frage: Aliphatische oder aromatische Nitrile, z. B. Acetonitril, Propionitril, Benzonitril, insbesondere Acetonitril, Ether, insbesondere cyclische Ether wie Dioxan, Tetrahydrofuran, insbesondere Dioxan.

Die Reaktionstemperatur kann bei dem erfindungsgemäßen Verfahren (d) in weiten Bereichen variiert werden. Im allgemeinen arbeitet man zwischen 50 °C und 200 °C, vorzugsweise bei 60 °C bis 150 °C.

Die Umsetzungen werden in der Regel bei Normaldruck ausgeführt, jedoch kann auch in geschlossenen Gefäßen gearbeitet werden, in denen sich dann in Abhängigkeit von der Art des Lösungsmittels und von der Temperatur ein entsprechend höherer Druck einstellt.

Die Reaktionspartner werden im allgemeinen im stöchiometrisch äquimolaren Verhältnis miteinander zur Reaktion gebracht. Man kann jedoch einen Überschuß an Isocyanat der Formel (VII) einsetzen, der bis zu 50 Mol-% betragen kann.

Bei der Durchführung des erfindungsgemäßen Verfahrens (d) wird das 2-Amino-4-halogen-5-nitrothiazol-Derivat der Formel (IV) in einem der genannten Lösungsmittel gelöst, das Isocyanat der Formel (VII) zugefügt und so lange auf die gewünschte Temperatur, vorzusweise auf die in dem betreffenden Lösungsmittel sich einstellende Rückflußtemperatur, erhitzt, bis die Umsetzung beendet ist. Die Reaktionszeiten

betragen je nach Reaktionspartner und Reaktionsbedingung im allgemeinen zwischen einer und hundert Stunden.

Die bei dem erfindungsgemäßen Verfahren (e) als Ausgangsstoffe benötigten 4-Halogen-2-halogencarbonylamino-5-nitrothiazol-Derivate der Formel (VIII) sind neu und ebenfalls Teil der Erfindung. Man erhält sie durch Umsetzung von 2-Amino-4-halogen-5-nitrothiazol-Derivaten der Formel (IV), wobei R die oben angegebene Bedeutung hat, mit der Ausnahme, daß R nicht Wasserstoff ist, mit Carbonyldihalogeniden der Formel (XI)

$O = C(Hal^4)_2$ (XI)

wobei

$Hal^4$ die oben angegebene Bedeutung hat,

in einem gegenüber den Reaktionspartnern inerten Lösungsmittel.

Verwendet man 2-Methylamino-4-chlor-5-nitrothiazol und Phosgen als Ausgangsstoffe, so kann der Verlauf des Verfahrens durch das folgende Formelschema wiedergegeben werden:

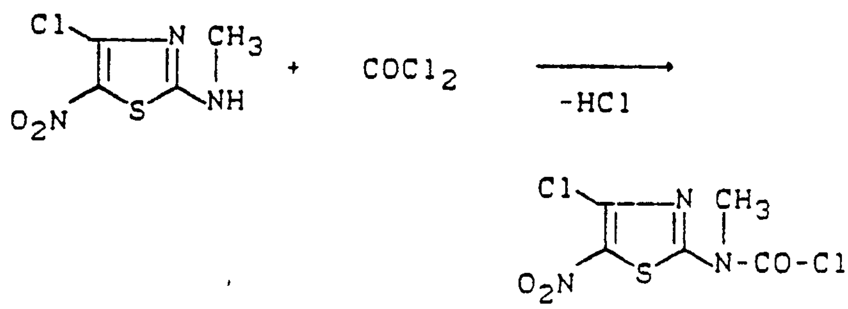


Als Lösungsmittel kommen bei der Durchführung des Verfahrens zur Herstellung der Vorprodukte der Formel (VIII) in Frage: Kohlenwasserstoffe wie Benzol, Methylbenzole, Halogenbenzole, ferner Ether, insbesondere cyclische Ether, insbesondere Dioxan.

Die Reaktionstemperatur kann bei dem Verfahren für die Vorprodukte der Formel (VIII) in weiten Bereichen variiert werden. Im allgemeinen arbeitet man zwischen 0 °C und 200 °C, vorzugsweise bei 50 °C bis 150 °C.

Die Umsetzungen werden in der Regel bei Normaldruck ausgeführt, jedoch kann auch in geschlossenen Gefäßen gearbeitet werden, in denen sich dann in Abhängigkeit von der Art des Lösungsmittels und von der Temperatur ein entsprechend höherer Druck einstellt.

Bei der Durchführung des Verfahrens zur Herstellung der Vorprodukte der Formel (VIII) wird das 2-Amino-4-halogen-5-nitrothiazol-Derivat der Formel (IV) mit einem der genannten Lösungsmittel versetzt und vorzugsweise bei der Rückflußtemperatur des Lösungsmittels so lange mit mindestens der stöchiometrisch erforderlichen Menge des Carbonyldihalogenids der Formel (XI), vorzugsweise mit Phosgen, behandelt, vorzugsweise unter Durchleiten durch die Reaktionsmischung, bis die Umsetzung zu (VIII) beendet ist. Der Verlauf der Umsetzung läßt sich leicht dünnschichtchromatographisch verfolgen. Die Reaktionszeiten betragen zwischen einer und hundert Stunden.

Zur Bindung des bei dem Verfahren zur Herstellung der Vorprodukte der Formel (VIII) gemäß Formelschema freiwerdenden Halogenwasserstoffs ist nicht in allen Fällen ein säurebindendes Mittel erforderlich. Falls säurebindende Mittel verwendet werden, kommen hierfür in Frage: Alkalihydroxide, -carbonate, -hydrogencarbonate oder Alkalihydride, z.B. Natriumhydrid.

Die bei dem erfindungsgemaßen Verfahren (e) weiterhin als Ausgangsstoffe benötigten Nucleophile der Formel (IX) sind grundsätzlich bekannt. Falls spezielle Verbindungen noch nicht beschrieben sind, lassen sie sich nach bekannten Verfahren herstellen.

Als Lösungsmittel kommen bei der Durchführung des erfindungsgemäßen Verfahrens (e) in Frage: Aliphatische oder aromatische Nitrile. z. B. Acetonitril. Propionitril, Benzonitril, insbesondere Acetonitril, Ether, insbeson dere cyclische Ether wie Dioxan. Tetrahydrofuran, insbesondere Dioxan.

Die Reaktionstemperatur kann bei dem erfindungsgemäßen Verfahren (e) in weiten Bereichen variiert werden. Im allgemeinen arbeitet man zwischen -20 °C und +150 °C, vorzugsweise bei 0 °C bis 100 °C.

Die Umsetzungen werden in der Regel bei Normaldruck ausgeführt, jedoch kann auch in geschlossenen Gefäßen gearbeitet werden, in denen sich dann in Abhängigkeit von der Art des Lösungsmittels und von der Temperatur ein entsprechend höherer Druck einstellt.

Die Reaktionspartner beim Verfahren (e) werden vorzugsweise im stöchiometrisch äquimolaren Verhältnis miteinander zur Reaktion gebracht. Im Falle von niederen aliphatischen Alkoholen als Nucleophile der Formel (IX) werden diese vorzugsweise gleichzeitig als Lösungsmittel eingesetzt. Im allgemeinen werden dann pro Gramm Verbindung der Formel (VIII) 5 bis 100 ml des aliphatischen Alkohols verwendet.

Zur Bindung des bei dem erfindungsgemäßen Verfahren (e) gemäß Formelschema freiwerdenden Halogenwasserstoffs ist nicht in allen Fällen - z.B. nicht bei aliphatischen Alkoholen - ein säurebindendes Mittel erforderlich. Falls säurebindende Mittel verwendet werden, kommen hierfür u.a. in Frage: Organische tertiäre Amine, z.B. Triethylamin oder Pyridin. Verwendet man als Nucleophil der Formel (IX) basisch wirkende primäre oder sekundäre Amine, so können diese gleichzeitig - indem man die doppelt stöchiometrische Menge einsetzt - als säurebindende Mittel verwendet werden. Ferner können anorganische säurebindende Mittel verwendet werden. Hierfür kommen in Frage: Alkalihydroxide, -carbonate, -hydrogencarbonate oder Alkalihydride, z.B. Natriumhydrid.

Bei der Durchführung des erfindungsgemäßen Verfahrens (e) wird das 4-Halogen-2-halogencarbonylamino-5-nitrothiazol-Derivat der Formel (VIII) in einem der genannten Lösungsmittel gelöst und das Nucleophil der Formel (IX) zugegeben. Dann wird - falls erforderlich - das säurebindende Mittel zugegeben und so lange in dem angegebenen Temperaturbereich gehalten, bis die Umsetzung beendet ist. Die Reaktionszeiten betragen je nach Reaktionspartner und Reaktionsbedingungen zwischen einer Minute und zehn Stunden.

Die erfindungsgemäßen Wirkstoffe der Formel (I) weisen eine starke Wirkung gegen Schädlinge auf und können zur Bekämpfung von unerwünschten Schadorganismen praktisch eingesetzt werden. Die Wirkstoffe sind für den Gebrauch als Pflanzenschutzmittel, u.a. als Fungizide, geeignet und außerdem auch für den Gebrauch zum Schutz technischer Materialien gegen Mikroben, wie z.B. Pilze.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden im Pflanzenschutz zur Bekämpfung von Pseudomonadaceae, Rhizobiaceae, Enterobacteriaceae, Corynebacteriaceae und Streptomycetaceae eingesetzt.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubense;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Dabei lassen sich die erfindungsgemäßen Wirkstoffe mit besonders gutem Erfolg zur Bekämpfung von

Obst-, Gemüse-und Getreidekrankheiten, wie z. B. gegen den Erreger des Apfelschorfes (Venturia inaequalis), gegen den Erreger der Braunfäule der Tomaten (Phytophthora infestans), gegen Erreger von Rebenkrankheiten (Plasmopara viticola) oder gegen Erreger von Getreidekrankheiten, wie z. B. Leptosphaeria nodorum, Cochliobolus sativus und Pyrenophora teres, einsetzen. Auch sei die Wirkung gegen Erreger von Reiskrankheiten (Pyricularia oryzae) und die gute in-vitro-Wirkung erwähnt.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemaßen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und anderen Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 40 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

Weiterhin sind die erfindungsgemäßen Wirkstoffe der Formel (I) auch zum Schutz technischer Materialien aufgrund ihres breiten Wirkungsspektrums geeignet.

Da technische Materialien von sehr vielen und sehr verschiedenen Mikrobenarten befallen und geschädigt werden können, ist ein breites Wirkungsspektrum, das auch vielfältige Verwendbarkeit ermöglicht, eine unabdingbare Eigenschaft fortschrittlicher Materialschutzstoffe.

Technische Materialien sind erfindungsgemäß nicht lebende Materialien, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Kühlkreisläufe genannt.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten), sowie gegen Bakterialien, Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puteana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Je nach Anwendungsgebiet kann ein erfindungsgemäßer Wirkstoff in die üblichen Formulierungen überführt werden, wie Lösungen, Emulsionen, Suspensionen, Pulver, Pasten und Granulate.

Diese können in an sich bekannter Weise hergestellt werden, z.B. durch Vermischen der Wirkstoffe mit einem Streckmittel, das aus flüssigem Lösungsmittel und/oder festen Trägerstoffen besteht, gegebenenfalls unter Verwendung von oberflächenaktiven Mitteln, wie Emulgatoren und/oder Dispergiermitteln, wobei gegebenenfalls im Falle der Benutzung von Wasser als Streckmittel organische Lösungsmittel wie Alkohole als Hilfsmittel verwendet werden können.

Flüssige Lösungsmittel für die Wirkstoffe können beispielsweise Wasser, Alkohole, wie niedere aliphatische Alkohole, vorzugsweise Ethanol oder Isopropanol, oder Benzylalkohol, Ketone, wie Aceton oder Methylethylketon, flüssige Kohlenwasserstoffe, wie Benzinfraktio nen, halogenierte Kohlenwasserstoffe, wie 1,2-Dichlorethan, sein.

Mikrobizide Mittel enthalten die Wirkstoffe im allgemeinen in einer Menge von 1 bis 95 %, bevorzugt von 10 bis 75 %.

Die Anwendungskonzentrationen der erfindungsgemäßen Wirkstoffe richten sich nach der Art und dem Vorkommen der zu bekämpfenden Mikroorganismen, sowie nach der Zusammensetzung des zu schützenden Materials. Die optimale Einsatzmenge kann durch Testreihen ermittelt werden. Im allgemeinen liegen die Anwendungskonzentrationen im Bereich von 0,001 bis 5 Gew.-%, vorzugsweise von 0,05 bis 1,0 Gew.-%, bezogen auf das zu schützende Material.

Die erfindungsgemaßen Wirkstoffe können auch in Mischung mit anderen bekannten Wirkstoffen vorliegen. Beispielsweise seien die folgenden Wirkstoffe genannt: Benzylalkoholmono(poly)hemiformal und andere Formaldehyd abspaltende Verbindungen, Benzimidazolyl-methylcarbamate, Tetramethylthiuramdisulfid, Zinksalze von Dialkyldithiocarbamaten, 2,4,5,6-Tetrachlorisophthalonitril, Thiazolylbenzimidazol, Mercaptobenzthiazol, 2-Rhodanidomethylthiobenzthiazol, Organo-Zinnverbindungen, Methylenbisthiocyanat, Phenolderivate, wie 2-Phenylphenol, (2,2′-Dihydroxy-5,5′-dichlor)-diphenylmethan und 3-Methyl-4-chlor-phenol, N-Trihalogenmethylthio-Verbindungen.

Die Herstellung und die Verwendung der erfindungsgemäßen Wirkstoffe geht aus den nachfolgenden Beispielen hervor.

## Herstellungsbeispiele

### Beispiel 1:

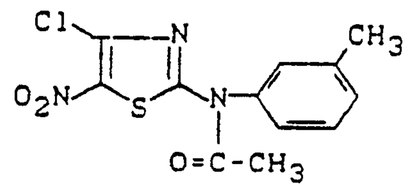

(Verbindung Nr. 42)

Eine Mischung aus 5,0 g (18,55 mmol) 4-Chlor-2-(3-methylphenylamino)-5-nitrothiazol (Schmelzpunkt 175 °C unter Zersetzung; Herstellung analog Beispiel A4) und 50 ml Acetanhydrid wird unter Rühren eine Stunde unter Rückfluß erhitzt. Anschließend werden im Rotationsverdampfer im Wasserstrahlvakuum das überschüssige Acetanhydrid und die gebildete Essigsäure abdestilliert, der Rückstand bei Raumtemperatur mit Wasser verrührt, abfiltriert und getrocknet. Man erhält 5,5 g (95,2 % der Theorie) 2-[N-Acetyl-N-(3-methylphenyl)]-amino-4-chlor-5-nitrothiazol vom Schmelzpunkt 137 °C bis 138 °C.

### Beispiel 2:

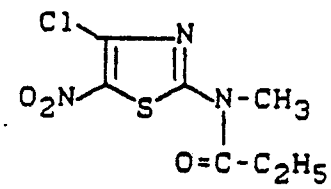

(Verbindung Nr. 4)

Eine Mischung aus 251,6 g (1,30 mol) 4-Chlor-2-methylamino-5-nitrothiazol (Schmelzpunkt 212 °C unter Zersetzung; Herstellung siehe Beispiel A8) und 1000 ml Propionsäureanhydrid wird unter Rühren eine Stunde unter Rückfluß erhitzt. Danach zeigt das Dünnschicht-Chromatogramm (Laufmittel Toluol/Essigsäureethylester 4:1), daß keine Ausgangsverbindung mehr vorhanden ist. Das Reaktionsgemisch wird anschließend unter Rühren auf etwa 8 °C gekühlt, der entstandene Niederschlag abfiltriert, mit wenig Propionsäureanhydrid, dann mit Petrolether gewaschen und getrocknet. Man erhält 254,3 g (78,4 % der Theorie) 2-(Propionyl-methylamino)-4-chlor-5-nitrothiazol vom Schmelzpunkt 132 °C bis 133 °C. Beim Verrühren der Propionsäureanhydrid-Mutterlauge mit 6 Liter Wasser über Nacht bei Raumtemperatur, anschließendes Abfiltrieren des entstandenen Niederschlages, Waschen desselben mit Wasser und Trocknen erhält man weitere 49,0 g (15,1 % der Theorie) 2-(Propionyl-methylamino)-4-chlor-5-nitrothiazol. Gesamtausbeute: 93,5 % der Theorie.

### Beispiel 3:

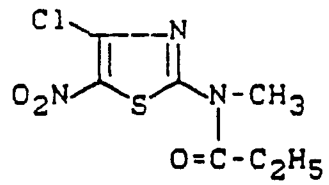

(Verbindung Nr. 4)

Eine Mischung aus 3,87 g (0,02 mol) 4-Chlor-2-methylamino-5-nitrothiazol (Schmelzpunkt 212 °C unter Zerset zung; Herstellung siehe Beispiel A8), 2,78 g (0,03 mol) Propionylchlorid und 50 ml Dioxan wird etwa 70 Stunden unter Rühren unter Rückfluß erhitzt. Nach dem Abkühlen wird in 500 ml Eiswasser verrührt, abfiltriert, mit Wasser gewaschen und getrocknet. Man erhält 4,60 g (92,2 % der Theorie) 2-(Propionyl-methylamino)-4-chlor-5-nitrothiazol, das in allen Eigenschaften identisch ist mit der nach Beispiel 2 erhaltenen Verbindung.

Beispiel 4:

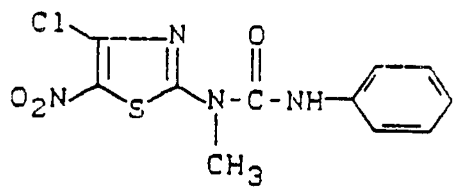

(Verbindung Nr. 70)

Eine Mischung aus 1935 mg (10 mmol) 4-Chlor-2-methylamino-5-nitrothiazol (Schmelzpunkt 212 °C unter Zersetzung; Herstellung siehe Beispiel A8), 1430 mg (12 mmol Phenylisocyanat und 50 ml Dioxan wird 22 Stunden unter Rühren unter Rückfluß erhitzt. Es wird auf Raumtemperatur abgekühlt, der entstandene Niederschlag abfiltriert, mit Dioxan gewaschen und getrocknet. Man erhält 1935 mg (61,9 % der Theorie) des Harnstoff-Derivats der oben angegebenen Formel vom Schmelzpunkt 225 °C unter Zersetzung.

Beispiel 5:

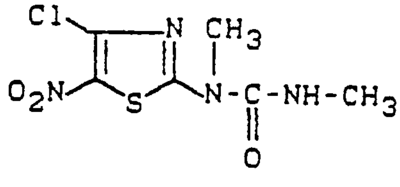

(Verbindung Nr. 2)

Eine Lösung von 60,0 g (0,302 mol) 2,4-Dichlor-5-nitrothiazol in 250 ml N-Methyl-2-pyrrolidinon wird mit 22,0 g (0,25 mol) N,N′-Dimethylharnstoff versetzt und unter Rühren etwa 20 Stunden auf 65 °C erwärmt. Danach wird auf Raumtemperatur abgekühlt und mit 2 Liter Eiswasser verrührt. Hierbei entsteht ein öliger Niederschlag, der nach Zugabe von etwa 200 ml Methylenchlorid in einem pulverigen Niederschlag übergeht. Er wird abfiltriert, mit Wasser gewaschen und getrocknet. Man erhält 18,9 g (25,0 % der Theorie)

des Harnstoff-Derivats der oben angegebenen Formel vom Schmelzpunkt 224° C bis 225° C unter Zersetzung. Bernsteingelbe Kristalle aus wenig Methanol.

Die identische Verbindung wird auch analog Beispiel 4 aus 4-Chlor-2-methylamino-5-nitrothiazol und Methylisocyanat in siedendem Dioxan erhalten.

Beispiel 6:

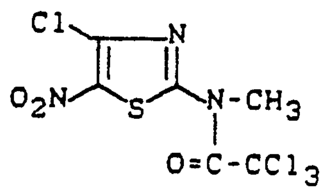

(Verbindung Nr. 15)

Eine Mischung aus 3,87 g (0,02 mol) 4-Chlor-2-methylamino-5-nitrothiazol (Schmelzpunkt 212° C unter Zersetzung; Herstellung siehe Beispiel A8), 4,55 g (0,025 mol) Trichloracetylchlorid und 50 ml Dioxan wird unter Rühren 2 Stunden unter Rückfluß erhitzt. Nach dem Abkühlen wird in 400 ml Eiswasser verruhrt, abfiltriert, mit Wasser gewaschen und getrocknet. Man erhält 6,50 g (95,9 % der Theorie) 4-Chlor-2-(methyl-trichloracetyl-amino)-5-nitrothiazol vom Schmelzpunkt 146° C bis 149° C unter Zersetzung.

Beispiel 7:

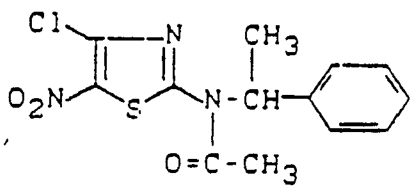

(Verbindung Nr. 101)

Eine Mischung aus 2,70 g (9,52 mmol) 4-Chlor-5-nitro-2-(1-phenethylamino)-thiazol (Schmelzpunkt 108° C aus Cyclohexan; Herstellung analog Beispiel A4) und 27 ml Acetanhydrid wird unter Rühren etwa 70 Stunden unter Rückfluß erhitzt. Die Aufarbeitung erfolgt analog Beispiel 1. Man erhält 1,95 g (62,9 %) der Verbindung der oben angegebenen Formel vom Schmelzpunkt 117° C bis 118° C unter Zersetzung.

Beispiel 8:

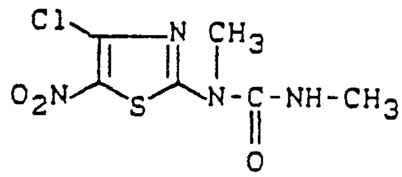

(Verbindung Nr. 2)

Zu einer Lösung von 1,02 g (4 mmol) 4-Chlor-2-(N-chlorcarbonyl-N-methyl)-amino-5-nitrothiazol (Herstellung siehe Beispiel A9) in 10 ml Dioxan wird bei etwa 15 °C eine Mischung aus 0,62 g (8 mmol) 40 %igem wäßrigem Methylamin und 5 ml Dioxan getropft, wobei sofort ein Niederschlag ausfällt. Nach 15-minütigem Nachrühren wird in 150 ml Eiswasser verruhrt, abfiltriert, mit Wasser gewaschen und getrocknet. Man erhält 0,96 g (96,2 % der Theorie) des Harnstoff-Derivats der oben angegebenen Formel, das in allen Eigenschaften identisch ist mit dem gemäß Beispiel 5 erhaltenen Produkt.

Beispiel 9:

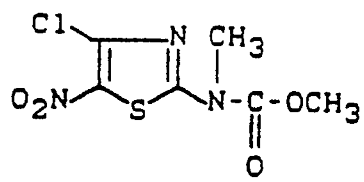

(Verbindung Nr. 104)

1,28 g (5 mmol) 4-Chlor-2-(N-chlorcarbonyl-N-methyl)-amino-5-nitrothiazol und 50 ml Methanol werden 10 Minu ten zum Sieden erhitzt. Anschließend wird im Wasserstrahlvakuum zur Trockne eingeengt. Es hinterbleiben 1,25 g (99 % der Theorie) des Carbaminsäure-Derivats der oben angegebenen Formel vom Schmelzpunkt 157 °C bis 158 °C.

Ein identisches Produkt wird erhalten, wenn man statt 10 Minuten zum Sieden zu erhitzen etwa eine Stunde bei Raumtemperatur kräftig verrührt.

Beispiel 10:

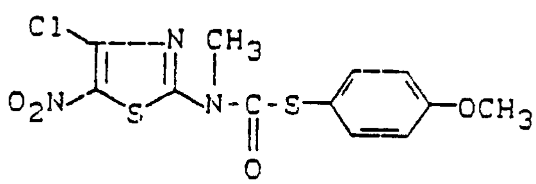

(Verbindung Nr. 105)

Zu einer Lösung von 1000 mg (3,9 mmol) 4-Chlor-2-(N-chlorcarbonyl-N-methyl)-amino-5-nitrothiazol (Herstellung siehe Beispiel A9) und 547 mg (3,9 mmol) 4-Mercaptoanisol in 20 ml Dioxan werden bei Raumtemperatur 395 mg (3,9 mmol) Triethylamin zugegeben, wobei sofort ein Niederschlag ausfällt. Nach einstündigem Nachrühren wird in etwa 200 ml Wasser verruhrt, abfiltriert, mit Wasser gewaschen und

getrocknet. Man erhält 1400 mg (99,7 % der Theorie) des Thiocarbaminsäure-Derivats der oben angegebenen Formel vom Schmelzpunkt 183° C bis 184° C.

Beispiel 11:

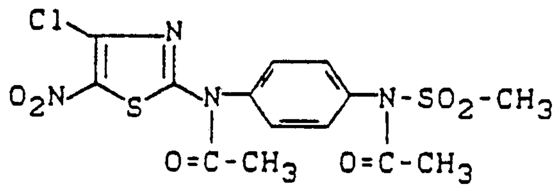

(Verbindung Nr. 85)

Eine Mischung aus 5,0 g (14,35 mmol) 4-Chlor-2-(4-methylsulfonylaminophenylamino)-5-nitrothiazol (Schmelzpunkt 203° C unter Zersetzung; Herstellung analog Beispiel A4) und 100 ml Acetanhydrid wird unter Rühren etwa 20 Stunden unter Rückfluß erhitzt. Anschließend wird im Wasserstrahlvakuum zur Trockne eingeengt und der Rückstand über Nacht zwischen zwei Tonplatten getrocknet. Man erhält 5,2 g (84 % der Theorie) des Diacetyl-Derivats der oben angegebenen Formel vom Schmelzpunkt 246° C bis 247° C unter Zersetzung.

Beispiel 12:

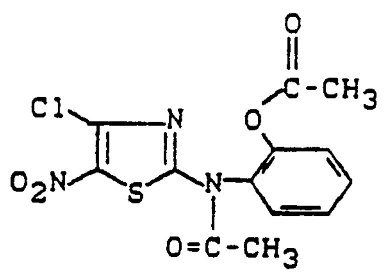

(Verbindung Nr. 6)

4-Chlor-2-(2-hydroxyphenylamino)-5-nitrothiazol (Schmelzpunkt 204° C; Herstellung analog Beispiel A4) wird analog Beispiel 1 umgesetzt. Man erhält in ebenfalls sehr guter Ausbeute das Diacetyl-Derivat der oben angegebenen Formel vom Schmelzpunkt 151° C.

Beispiel 13:

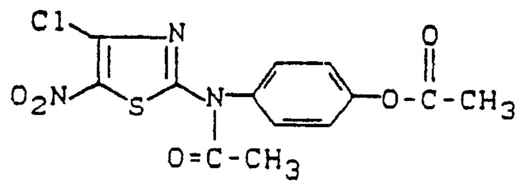

(Verbindung Nr. 62)

4-Chlor-2-(4-hydroxyphenylamino)-5-nitrothiazol (Schmelzpunkt 174 °C bis 176 °C unter Zersetzung; Herstellung analog Beispiel A4) wird analog Beispiel 1 umgesetzt. Man erhält in ebenfalls sehr guter Ausbeute das Diacetyl-Derivat der oben angegebenen Formel vom Schmelzpunkt 197 °C.

Beispiel 14:

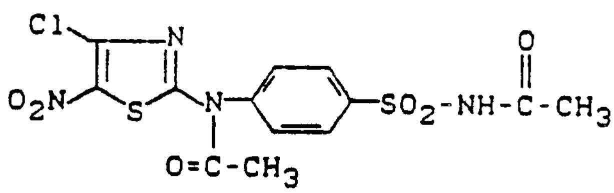

(Verbindung Nr. 91)

2-(4-Aminosulfonylphenylamino)-4-chlor-5-nitrothiazol (Schmelzpunkt oberhalb 280 °C; Herstellung analog Beispiel 4) wird analog Beispiel 1 umgesetzt. Man erhält in sehr guter Ausbeute das Diacetyl-Derivat der oben angegebenen Formel vom Schmelzpunkt 244 °C unter Zersetzung.

Beispiel 15:

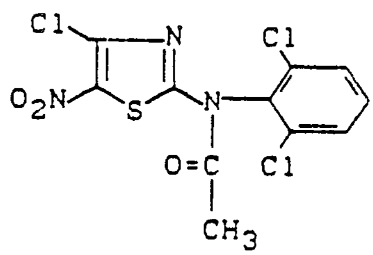

(Verbindung Nr. 103)

4-Chlor-2-(2.6-dichlorphenylamino)-5-nitrothiazol (Schmelzpunkt 141 °C; Herstellung siehe Beispiel A10) wird analog den Angaben in Beispiel 1 umgesetzt nur mit dem Unterschied, daß statt einer Stunde 40 Stunden unter Rückfluß erhitzt wird. Danach ist nach dünnschichtchromatographischer Analyse kein Ausgangsprodukt mehr vorhanden. Nach Aufarbeitung analog Beispiel 1 erhält man in ebenfalls sehr guter Ausbeute 2-(N-Acetyl-N-2,6-dichlorphenyl)-amino-4-chlor-5-nitrothiazol vom Schmelzpunkt 95 °C.

Beispiel 16:

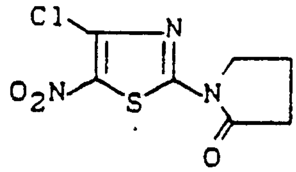

(Verbindung Nr. 107)

Eine Lösung von 19,9 g (0,1 mol) 2,4-Dichlor-5-nitrothiazol und 4,25 g (0,05 mol) Pyrrolidinon-(2) in 150 ml Tetrahydrofuran wird unter Rühren bei etwa 0-5 °C portionsweise mit 1,8 g (0,06 mol) Natriumhydrid (80 %ig in Paraffin) versetzt. Nach vierstündigem Nachrühren bei Raumtemperatur wird in 1 l Eiswasser eingerührt und mit Salzsäure angesäuert. Man dekantiert vom halbfesten Niederschlag ab und verrührt diesen intensiv mit 500 ml Petrolether. Danach wird der nun pulverige Niederschlag abfiltriert, mit Petrolether nachgewaschen und getrocknet. Das Rohprodukt (9,42 g) wird durch Sublimation bei 150 °C/0,1 mbar gereinigt. Man erhält 2,05 g (16,6 % der Theorie) des Pyrrolidinon-Derivats der oben angegebenen Formel vom Schmelzpunkt 172,5 °C bis 173 °C (aus Cyclohexan).

Beispiel 17:

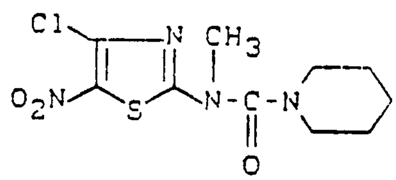

(Verbindung Nr. 108)

Zu einer Lösung von 1,28 g (0,004 mol) 4-Chlor-2-(N-chlorcarbonyl-N-methyl)-amino-5-nitrothiazol in 200 ml Dioxan werden bei Raumtemperatur unter Rühren 0,85 g (0,01 mol) Piperidin getropft, wobei sofort ein Niederschlag ausfällt. Nach 15-minütigem Nachrühren wird in 200 ml Wasser eingerührt, abfiltriert, mit Wasser gewaschen und getrocknet. Man erhält 1,48 g (97,4 % der Theorie) des Harnstoffs der oben angegebenen Formel. Der Schmelzpunkt nach Umkristallisation aus Cyclohexan ist 168 °C bis 171 °C.

Beispiel 18:

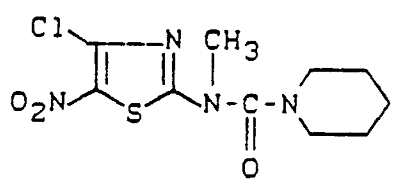

(Verbindung Nr. 108)

Eine Lösung von 840 mg (4,34 mmol) 4-Chlor-2-methylamino-4-nitrothiazol in 200 ml Acetonitril wird mit 960 Mg (6,51 mmol) N-Chlorcarbonylpiperidin und 899 mg (6,51 mmol) Kaliumcarbonat versetzt. Nach

Rühren bei Raumtemperatur über Nacht wird mit 1 l Wasser verrührt, mit Salzsäure angesäuert, abfiltriert, mit Wasser gewaschen und getrocknet. Man erhält 850 mg (64,3 % der Theorie) des Harnstoffs der oben angegebenen Formel, der identisch ist mit dem nach Beispiel 17 erhaltenen Produkt. Die Verbindung kann statt durch Umkristallisation aus Cyclohexan auch durch Sublimation bei 130° C/0,1 mbar gereinigt werden.

Herstellung bisher nicht literaturbekannter Ausgangsprodukte

Beispiel A1

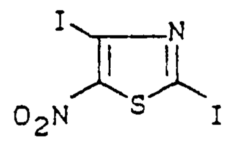

19,9 g (0,1 mol) 5,4-Dichlor-5-nitrothiazol werden in 500 ml Aceton gelöst und nach Zugabe von 150 g (1 mol) Natriumiodid wird etwa 60 Stunden unter Rückfluß gerührt. Danach liegt der Anteil an 2,4-Diiod-5-nitrothiazol im Reaktionsgemisch nach der gaschromatographischen Analyse bei über 95 %. Daraufhin wird das Lösungsmittel im Vakuum abgezogen, der Rückstand in Wasser verrührt, abfiltriert, mit Wasser gewaschen und getrocknet. Man erhält 33,9 g (89 % der Theorie) 2,4-Diiod-5-nitrothiazol vom Schmelzpunkt 135° C nach dem Umkristallisieren aus Cyclohexan.

Beispiel A2

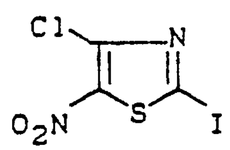

und

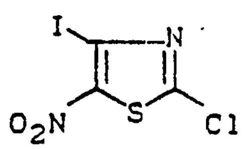

39,8 g (0,2 mol) 2,4-Dichlor-5-nitrothiazol werden in 500 ml Aceton gelöst und nach Zugabe von 90 g (0,6 mol) Natriumiodid wird etwa 6 Tage bei Raumtemperatur gerührt, wobei der Fortgang der Umsetzung gaschromatographisch verfolgt wird. Dann wird das Lösungsmittel im Vakuum abgezogen, der Rückstand in Wasser verrührt, abfiltriert, mit Wasser gewaschen und getrocknet. Man erhält ein Substanzgemisch, das nach der gaschromatographischen Analyse 71,5 % 2-Iod-4-chlor-5-nitrothiazol und 25,8 % 2-Chlor-4-iod-5-nitrothiazol enthält. HPLC-(= High pressure liquid chromatography) chromatographische Trennung lieferte die Reinsubstanzen mit den folgenden Schmelzpunkten:

2-Iod-4-chlor-5-nitrothiazol: Schmelzpunkt 80° C - 81° C,

2-Chlor-4- iod-5-nitrothiazol: Schmelzpunkt 108° C -109,5° C.

Beispiel A3

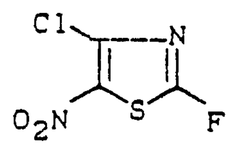

19,9 g (0,1 mol) 2,4-Dichlor-5-nitrothiazol werden in 60 ml Acetonitril gelöst und nach Zugabe von 24 g

(0,4 mol) geglühtem Kaliumfluorid und 0,5 g [18]Krone-6 wird etwa eine Woche bei Raumtemperatur gerührt, wobei der Fortgang der Umsetzung gaschromatographisch verfolgt wird. Der Rohansatz wird bei 0,1 bis 0,2 mbar und bei einer Temperatur von 20 bis 25° C destilliert, wobei die flüchtigen Bestandteile in einer mit Methanol/Trockeneis gekühlten Vorlage aufgefangen werden. Das Acetonitril wird anschließend bei gewöhnlichem Druck abdestilliert, wobei ein Gemisch aus etwa gleichen Teilen 4-Chlor-2-fluor-5-nitrothiazol und 2,4-Dichlor-5-nitrothiazol zurückbleibt. Hieraus läßt sich das 4-Chlor-2-fluor-5-nitrothiazol zum Beispiel durch fraktionierte Destillation abtrennen.

$^{19}$F-NMR ($CDCl_3$) ($CF_3$-COOH als externer Standard): $\delta$ = -17,5 ppm.

MS: 182 (37%) = $M^+$ = $C_3ClFN_2O_2S$

124 (34%)

91 (60%)

63 (100%)

Als Nebenkomponente in einem relativen Anteil zwischen 1 und 5% bezogen auf 4-Chlor-2-fluor-5-nitrothiazol kann durch Gaschromatographie das isomere 2-Chlor-4-fluor-5-nitrothiazol identifiziert werden.

$^{19}$F-NMR ($CDCl_3$) ($CF_3$-COOH als externer Standard): $\delta$ = - 22,7 ppm.

MS: 182 (83%) = $M^+$ = $C_3ClFN_2O_2S$

136 (32%)

91 (43%) 75 (100%)

Beispiel A4

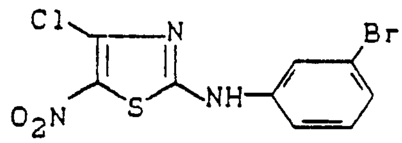

Zu einer Lösung von 12,0 g (0,06 mol) 2,4-Dichlor-5-nitrothiazol in 100 ml Dimethylformamid tropft man bei 0° C bis 5° C im Verlauf von etwa 2 Stunden eine Lösung von 8,6 g (0,05 mol) 3-Bromanilin in 100 ml Dimethylformamid zu und rührt noch 4 Stunden bei derselben Temperatur nach. Dann wird in 1000 ml Eiswasser eingerührt, der Niederschlag abfiltriert, mit Wasser gewaschen und getrocknet. Zur Entfernung von überschüssigem 2,4-Dichlor-5-nitrothiazol wird bei Raumtemperatur in 80 ml Petrolether verrührt, abfiltriert, mit Petrolether gewaschen und getrocknet. Man erhält so 16,0 g (95,7 % der Theorie) 2-(3-Bromphenylamino)-4-chlor-5-nitrothiazol vom Schmelzpunkt 191° C bis 192° C (Zers.)

Beispiel A5

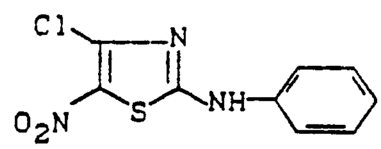

Eine Lösung von 360 g (1,8 mol) 2,4-Dichlor-5-nitrothiazol in 1,5 l Acetonitril wird mit 252 g (3,0 mol) Natriumhydrogencarbonat versetzt. Anschließend tropft man bei - 10° C bis - 15° C im Verlauf von etwa 4,5 Stunden eine Mischung aus 139,5 g (1,5 mol) Anilin und 1.500 ml Acetonitril zu und rührt weitere 4 Stunden bei derselben Temperatur nach. Dann wird in 15 l Eiswasser eingerührt, der Niederschlag abfiltriert, mit Wasser gewaschen und getrocknet. Zur Entfernung von überschüssigem 2,4-Dichlor-5-nitrothiazol wird bei Raumtemperatur in 2,4 l Petrolether verrührt, abfiltriert, mit Petrolether gewaschen und getrocknet. Man erhält 361,7 g (94,4 % der Theorie) 2-Anilino-4-chlor-5-nitrothiazol vom Schmelzpunkt 186° C bis 187° C (Zers.)

Beispiel A6

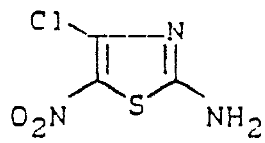

Eine Lösung von 19,9 g (0,1 mol) 2,4-Dichlor-5-nitrothiazol in 200 ml Acetonitril wird unter Rühren mit 10,1 g (0,105 mol) Ammoniumcarbonat versetzt und zwei Tage bei Raumtemperatur weitergerührt. Anschließend wird in 1 l Eiswasser eingerührt, der Niederschlag abfiltriert, mit Wasser gewaschen und getrocknet. Zur Entfernung von nicht umgesetztem 2,4-Dichlor-5-nitrothiazol wird bei Raumtemperatur in etwa 100 ml Petrolether verrührt, abfiltriert, mit Petrolether gewaschen und getrocknet. Man erhält 12,2 g (68,0 % der Theorie) 2-Amino-4-chlor-5-nitrothiazol vom Schmelzpunkt 180 °C (Zers.).

Beispiel A7

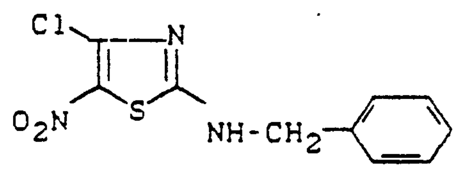

Zu einer Lösung von 12,0 g (0,06 mol) 2,4-Dichlor-5-nitrothiazol in 50 ml Acetonitril werden 7,0 g (0,051 mol) wasserfreies Kaliumcarbonat gegeben. Anschließend tropft man bei 15 °C bis 20 °C im Verlauf von etwa einer halben Stunde eine Mischung aus 5,35 g (0,05 mol) Benzylamin und 50 ml Acetonitril zu und rührt eine weitere Stunde bei Raumtemperatur nach. Anschließend wird 15 Minuten auf Rückflußtemperatur erhitzt, auf Raumtemperatur abgekühlt, in etwa 500 ml Eiswasser eingerührt, der Niederschlag abfiltriert, mit Wasser gewaschen und getrocknet. Zur Entfernung von überschüssigem 2,4-Dichlor-5-nitrothiazol wird bei Raumtemperatur in 80 ml Petrolether verrührt, abfiltriert, mit Petrolether gewaschen und getrocknet. Man erhält 12,9 g (95,7 % der Theorie) 2-Benzylamino-4-chlor-5-nitrothiazol vom Schmelzpunkt 195 °C bis 196 °C (Zers.).

Beispiel A8

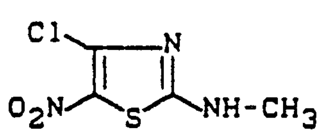

Zu einer Lösung von 398 g (2.0 mol) 2,4-Dichlor-5-nitrothiazol in 2500 ml Acetonitril tropft man langsam bei -10 °C bis 0 °C 315,3 g (3.0 mol) 29,5 %ige wäßrige Methylamin-Lösung. Nach dem Weiterrühren über Nacht bei Raumtemperatur wird in 15 Liter Eiswasser eingerührt, der Niederschlag abfiltriert, mit Wasser gewaschen und getrocknet. Nach Verrühren mit etwa 1,5 Liter Petrolether bei Raumtemperatur wird abfiltriert, mit Petrolether gewaschen und getrocknet. Man erhält 289,0 g (74,7 % der Theorie) 4-Chlor-2-methylamino-5-nitrothiazol vom Schmelzpunkt 212 ° C (Zers .). Die Verbindung kann aus Methanol umkristallisiert werden und ist bei 140 °C/0,1 mbar sublimierbar.

Beispiel A9

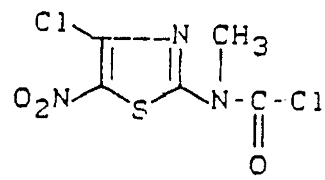

In eine siedende Suspension von 19,4 g (0,1 mol) 4-Chlor-2-methylamino-5-nitrothiazol in 350 ml Dioxan (90-95 °C) wird ein kräftiger Phosgenstrom geleitet. Nach etwa 15 Minuten erhält man eine klare Lösung. Nach weiterem etwa zweistündigem Phosgeneinleiten wird mit trockenem Stickstoff ausgeblasen und im Wasserstrahlvakuum bei 40 °C Badtemperatur eingeengt. Es hinterbleiben 25,4 g (99 % der Theorie) 4-Chlor-2-(N-chlorcarbonyl-N-methyl)-amino-5-nitrothiazol. Schmelzpunkt nach Umkristallisieren aus Cyclohexan: 130 °C bis 131 °C.

Beispiel A10

a) 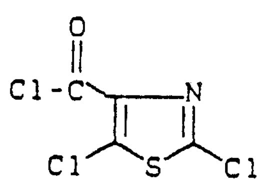

(Ausgangsprodukt für Verbindung Nr. 19)

Eine Mischung aus 143,5 g (0,725 mol) 2,5-Dichlorthiazol-4-carbonsäure und 700 ml Thionylchlorid wird unter Rühren langsam erhitzt. Bereits bei etwa 40 °C tritt starke Gasentwicklung ein. Im Verlauf einer halben Stunde wird bis auf Rückflußtemperatur erhitzt und dort bis zum Ende der Gasentwicklung gehalten (ca. 2 Stunden): Endtemperatur etwa 80 °C.

Nach dem Abziehen des überschüssigen Thionylchlorids im Wasserstrahlvakuum hinterbleiben 144,6 g (92,2 % der Theorie) kristallines 4-Chlorcarbonyl-2,5-dichlor-thiazol. Aus Petrolether große, farblose Kristalle vom Schmelzpunkt 58-59 °C.

b) 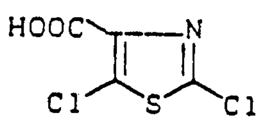

271,5 g (1 mol) 2,5-Dichlor-4-trichlormethylthiazol und 2700 ml Wasser werden unter Rühren über Nacht (etwa 15 Stunden) unter Rückfluß erhitzt. Nach dem Abkühlen nuf Raumtemperatur wird der entstandene kristalline Niederschlag abfiltriert, mit Wasser gewaschen und getrocknet.

Ausbeute 143,5 g (72,5 % der Theorie) 2,5-Dichlorthiazol-4-carbonsäure. Die Verbindung ist bei 120 °C/0,1 mbar sublimierbar und z.B. aus Chloroform umkristallisierbar. Schmelzpunkt 191 °C unter Zersetzung. Durch Einengen der wäßrigen Phase können weitere 30,5 g etwas weniger reines Produkt isoliert werden.

c)

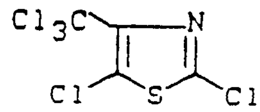

In einem Dreihalskolben, der mit Rührer, Thermometer, Rückflußkühler und Gaseinleitungsrohr versehen ist, wird beginnend bei Raumtemperatur in eine Mischung aus 1093 g (8,19 mol) 2-Chlor-4-methylthiazol und 4 l Methylenchlorid Chlorgas eingeleitet. Nach Abklingen der exothermen Reaktion wird unter allmählicher Temperatursteigerung und weiterem Einleiten von Chlor zunächst das Methylenchlorid abdestilliert und dann der Sumpf langsam bis auf etwa 160 °C erhitzt. Bei etwa 160 °C wird dann so lange meist überschüssiges Chlorgas (erkennbar an der leicht grünlichen Farbe des Abgases) eingeleitet, bis im Gaschromatogramm fast nur noch die gewünschte Verbindung 2,5-Dichlor-4-trichlormethylthiazol zu erkennen ist. Gesamtdauer der Chlorierung 40 bis 50 Stunden.

Eine Grobdestillation bis zu einer Kopftemperatur von 150 °C bei 14 mbar liefert 2057 g ca. 95 %iges 2,5-Dichlor-4-trichlormethyl-thiazol, was einer Ausbeute von 88 % der Theorie bezogen auf reines Produkt entspricht. Das 2,5-Dichlor-4-trichlormethyl-thiazol wird durch eine Feindestillation an einer ca. 220 cm langen, silberverspiegelten Füllkörperkolonne rein erhalten. Siedepunkt 123 °C bis 125 °C bei 16 mbar.

Beispiel A11

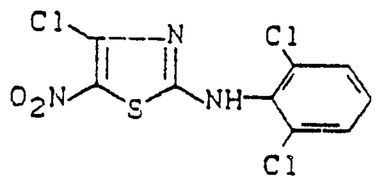

Eine Mischung aus 6,48 g (0,04 mol) 2,6-Dichloranilin, 1,4 g (0,047 mol) Natriumhydrid (80 %ig in Paraffin) und 50 ml Tetrahydrofuran wird 2 Stunden bei Raumtemperatur gerührt. Anschließend wird die Suspension im Verlauf einer Stunde zu einer Lösung aus 15,92 g (0,08 mol) 2,4-Dichlor-5-nitrothiazol in 100 ml Tetrahydrofuran getropft. Nach etwa dreistündigem Nachrühren bei Raumtemperatur wird auf 600 ml Wasser gegeben, das Rohprodukt mit Methylenchlorid extrahiert, die Methylenchloridphase getrocknet und eingeengt. Der Rückstand wird bei 135 °C/0,1 mbar sublimiert und das Sublimat aus Petrolether umkristallisiert. Man erhält 1,2 g (9,2 % der Theorie) 4-Chlor-2-(2,6-dichlorphenylamino)-5-nitrothiazol vom Schmelzpunkt 141 °C.

In entsprechender Weise und gemäß den allgemeinen Angaben zur Herstellung erhält man die folgenden 2-Acylamino-4-halogen-5-nitrothiazol-Derivate der allgemeinen Formel (I).

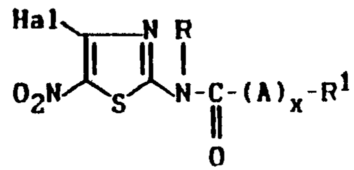

(I)

| Verb. Nr. | Endprodukte Hal | -R | $-(A)_x-R^1$ | Schmp. (°C) (umkrist. aus) | Herstellung | Ausgangsverbindung Schmp. (°C) | Herstellung |
|---|---|---|---|---|---|---|---|
| 1 | Cl | 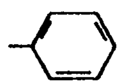 | $-CH_3$ | 176-7 | analog Beispiel 1 | 186-7 (Z.) | siehe Beispiel A5 |
| 2 | Cl | $-CH_3$ | $-NH-CH_3$ | 224-5 (Z.) | siehe Beispiel 5 und siehe Beispiel 8 | 212 (Z.)<br>130-1 | siehe Bsp. A8<br>siehe Bsp. A9 |
| 3 | Cl | $-CH_3$ | $-CH_3$ | 178-9 | analog Beispiel 2 | 212 (Z.) | siehe Beispiel A8 |
| 4 | Cl | $-CH_3$ | $-C_2H_5$ | 132-3 | siehe Beispiele 2 und 3 | 212 (Z.) | siehe Beispiel A8 |
| 5 | Cl | 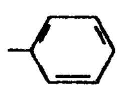 | $-C_2H_5$ | 151-4 | analog Beispiel 1 | 186-7 (Z.) | siehe Beispiel A5 |
| 6 | Cl | 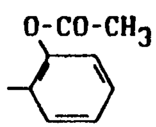 | $-CH_3$ | 151 | siehe Beispiel 12 | 204 | analog Beispiel A4 |

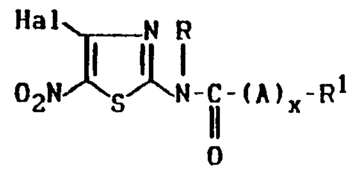

(I)

| Verb. Nr. | Endprodukte Hal | -R | $-(A)_x-R^1$ | Schmp. (°C) (umkrist. aus) | Herstellung. | Ausgangsverbindung Schmp. (°C) | Herstellung |
|---|---|---|---|---|---|---|---|
| 7 | Cl | H | $-CH_3$ | 198-202 | analog Beispiel 2 | 180 (Z.) | siehe Beispiel A6 |
| 8 | Cl | 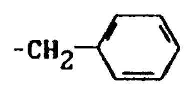 | $-CH_3$ | 174-5 (Z.) | analog Beispiel 1 | 195-6 (Z.) | siehe Beispiel A7 |
| 9 | Cl | 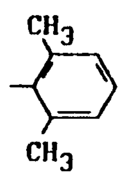 | $-CH_3$ | 227 (Z.) | analog Beispiel 1 | 215-6 (Z.) | analog Beispiel A4 |
| 10 | Cl | 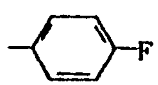 | $-CH_3$ | 204 (Z.) | analog Beispiel 1 | 112-3 (Z.) | analog Beispiel A4 |
| 11 | Cl | 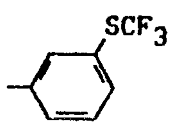 | $-CH_3$ | 147-8 (Z.) | analog Beispiel 1 | 77-8 | analog Beispiel A4 |
| 12 | Cl | 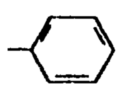 | $-CH_2Cl$ | 152-4 (Z.) | analog Beispiel 3 | 186-7 (Z.) | siehe Beispiel A5 |

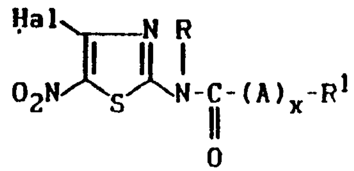

(I)

| Verb. Nr. | Endprodukte Hal | -R | $-(A)_x-R^1$ | Schmp. (°C) (umkrist. aus) | Herstellung | Ausgangsverbindung Schmp. (°C) | Herstellung |
|---|---|---|---|---|---|---|---|
| 13 | Cl | $-CH_3$ | $-CH_2Cl$ | 190-190,5 | analog Beispiel 3 | 212 (Z.) | siehe Beispiel A8 |
| 14 | Cl | $-CH_3$ | 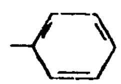 | 168 | analog Beispiel 3 | 212 (Z.) | siehe Beispiel A8 |
| 15 | Cl | $-CH_3$ | $-CCl_3$ | 146-9 (Z.) | siehe Beispiel 6 | 212 (Z.) | siehe Beispiel A8 |
| 16 | Cl | 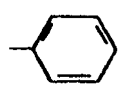 | $-CHCl_2$ | 167-9 (Cyclohexan) | analog Beispiel 3 | 186-7 (Z.) | siehe Beispiel A5 |
| 17 | Cl | $-CH_3$ | $-CHCl_2$ | 196-196,5 | analog Beispiel 6 | 212 (Z.) | siehe Beispiel A8 |
| 18 | Cl | $-CH_3$ | 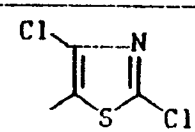 | 197 | analog Beispiel 3 | 212 (Z.) | siehe Beispiel A8 |
| 19 | Cl | $-CH_3$ | 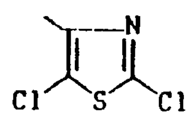 | 183-4 (Z.) | analog Beispiel 3 | 212 (Z.) | siehe Beispiel A8 |

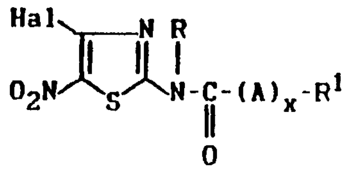

(I)

| Verb. Nr. | Endprodukte Hal | -R | $-(A)_x-R^1$ | Schmp. (°C) (umkrist. aus) | Herstellung | Ausgangsverbindung Schmp. (°C) | Herstellung |
|---|---|---|---|---|---|---|---|
| 20 | Cl | H | $-CH_2Cl$ | 158-64 (Z.) (Toluol) | analog Beispiel 3 | 180 (Z.) | siehe Beispiel A6 |
| 21 | Cl | H | $-CHCl_2$ | 138-40 | analog Beispiel 6 | 180 (Z.) | siehe Beispiel A6 |
| 22 | Cl | H | $-CCl_3$ | 112-4 | analog Beispiel 6 | 180 (Z.) | siehe Beispiel A6 |
| 23 | Cl | $-CH_3$ | 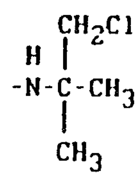 | 153-5 (Z.) (Toluol) | analog Beispiel 4 | 212 (Z.) | siehe Beispiel A8 |
| 24 | Cl | $-CH_3$ | $-CF_3$ | 127-9 | analog Beispiel 2 | 212 (Z.) | siehe Beispiel A8 |
| 25 | Cl | H | $-CF_3$ | 155-6 (Z.) | analog Beispiel 2 | 180 (Z.) | siehe Beispiel A6 |
| 26 | Cl | 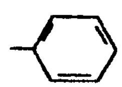 | $-CCl_3$ | 104 (Z.) | analog Beispiel 3 | 186-7 (Z.) | siehe Beispiel A5 |

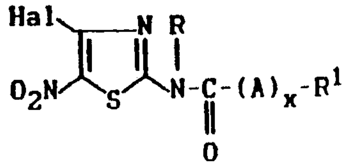

(I)

| Verb. Nr. | Endprodukte Hal | -R | $-(A)_x-R^1$ | Schmp. (°C), (umkrist. aus) | Herstellung | Ausgangsverbindung Schmp. (°C) | Herstellung |
|---|---|---|---|---|---|---|---|
| 27 | Cl | $-CH_3$ | $-CH_2-C_2H_5$ | 114-5 | analog Beispiel 3 | 212 (Z.) | siehe Beispiel A8 |
| 28 | Cl | $-CH_3$ | 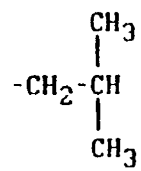 | 94-6 | analog Beispiel 3 | 212 (Z.) | siehe Beispiel A8 |
| 29 | Cl | $-C_2H_5$ | $-CH_2Cl$ | 139-42 | analog Beispiel 3 | 167-8 (Z.) | analog Beispiel A8 |
| 30 | Cl | $-CH_2-C_2H_5$ | $-CH_2Cl$ | 117-21 | analog Beispiel 3 | ab 106 (Z.) | analog Beispiel A8 |
| 31 | Cl | $-CH_2-CH_2-OCH_3$ | $-CH_2Cl$ | 156-9 | analog Beispiel 3 | 124-8 | analog Beispiel A8 |
| 32 | Cl | H | 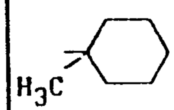 | 152-4 | analog Beispiel 3 | 180 (Z.) | siehe Beispiel A6 |
| 33 | Cl | $-CH_2-CH_2Cl$ | $-CH_2Cl$ | 111,5-115 | analog Beispiel 3 | 143-4 (Z.) | analog Beispiel A8 |

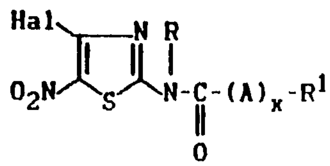

(I)

| Verb. Nr. | Endprodukte Hal | -R | -(A)$_x$ $R^1$ | Schmp.($^0$C) (umkrist. aus) | Herstellung | Ausgangsverbindung Schmp.($^0$C) | Herstellung |
|---|---|---|---|---|---|---|---|
| 34 | Cl | $-C_2H_5$ | $-C_2H_5$ | 119,5-121 | analog Beispiel 3 | 167-8 (Z.) | analog Beispiel A8 |
| 35 | Cl | $-CH_2-C_2H_5$ | $-C_2H_5$ | 99,5-100,5 | analog Beispiel 3 | 167-8 (Z.) | analog Beispiel A8 |
| 36 | Cl | $-CH_2-CH_2-OCH_3$ | $-C_2H_5$ | 101,5-103 | analog Beispiel 3 | 124-8 | analog Beispiel A8 |
| 37 | Cl | 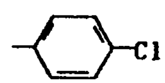 | $-CH_3$ | 187 | analog Beispiel 1 | 207 (Z.) | analog Beispiel A4 |
| 38 | Cl | 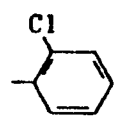 | $-CH_3$ | 152 | analog Beispiel 1 | 151 (Z.) | analog Beispiel A4 |
| 39 | Cl | 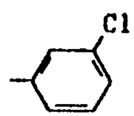 | $-CH_3$ | 176 (Z.) | analog Beispiel 1 | 197 (Z.) | analog Beispiel A4 |
| 40 | Cl | 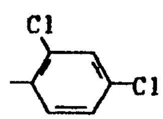 | $-CH_3$ | 197-8 | analog Beispiel 1 | 188-9 (Z.) | analog Beispiel A4 |

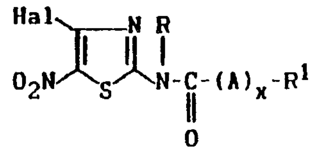

(I)

| Verb. Nr. | Endprodukte Hal | -R | $-(A)_x-R^1$ | Schmp. (°C) (umkrist. aus) | Herstellung | Ausgangsverbindung Schmp. (°C) | Herstellung |
|---|---|---|---|---|---|---|---|
| 41 | Cl | 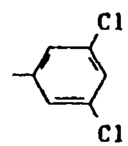 | $-CH_3$ | 245 (Z.) | analog Beispiel | 142-4 (Z.) | analog Beispiel A4 |
| 42 | Cl | 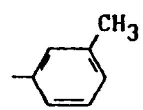 | $-CH_3$ | 137-8 | siehe Beispiel 1 | 175 (Z.) | analog Beispiel A4 |
| 43 | Cl | 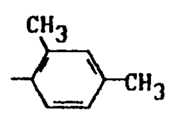 | $-CH_3$ | 167-8 | analog Beispiel 1 | 177-8 (Z.) | analog Beispiel A4 |
| 44 | Cl | 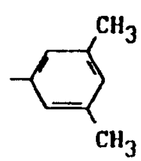 | $-CH_3$ | 207 | analog Beispiel 1 | 179-80 (Z.) | analog Beispiel A4 |
| 45 | Cl | 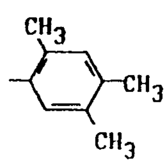 | $-CH_3$ | 169-70 | analog Beispiel 1 | 183-5 (Z.) | analog Beispiel A4 |

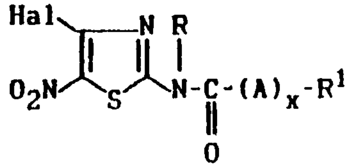 (I)

| Verb. Nr. | Endprodukte Hal | -R | $-(A)_x-R^1$ | Schmp. (°C) (umkrist. aus) | Herstellung | Ausgangsverbindung Schmp. (°C) | Herstellung |
|---|---|---|---|---|---|---|---|
| 46 | Cl | 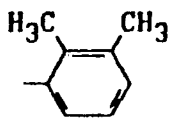 | $-CH_3$ | 192-3 (Z.) | analog Beispiel 1 | 202 (Z.) | analog Beispiel A4 |
| 47 | Cl | 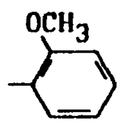 | $-CH_3$ | 176 | analog Beispiel 1 | 185-6 (Z.) | analog Beispiel A4 |
| 48 | Cl | 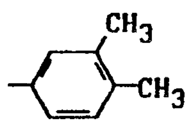 | $-CH_3$ | 198 (Z.) | analog Beispiel 1 | 178-9 (Z.) | analog Beispiel A4 |
| 49 | Cl | 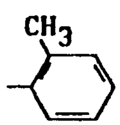 | $-CH_3$ | 156-7 | analog Beispiel 1 | 180-1 (Z.) | analog Beispiel A4 |
| 50 | Cl | 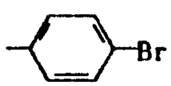 | $-CH_3$ | 236 (Z.) | analog Beispiel 1 | 208 (Z.) | Beispiel A4 |

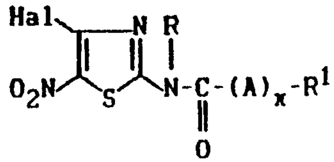

(I)

| Verb. Nr. | Endprodukte Hal | -R | $-(A)_x-R^1$ | Schmp. (°C) (umkrist. aus) | Herstellung | Ausgangsverbindung Schmp. (°C) | Herstellung |
|---|---|---|---|---|---|---|---|
| 51 | Cl | 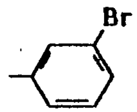 | $-CH_3$ | 162 | analog Beispiel 1 | 191-2 (Z.) | Beispiel A4 |
| 52 | Cl | 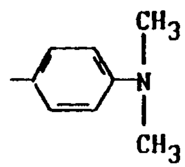 | $-CH_3$ | 151 | analog Beispiel 1 | >260 | analog Beispiel A4 |
| 53 | Cl | 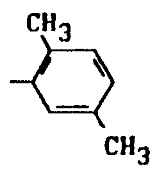 | $-CH_3$ | 158 | analog Beispiel 1 | 186-7 (Z.) | analog Beispiel A4 |
| 54 | Cl | 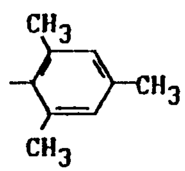 | $-CH_3$ | 203 | analog Beispiel 1 | 214-5 (Z.) | analog Beispiel A4 |
| 55 | Cl | 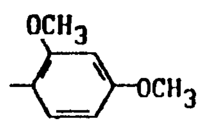 | $-CH_3$ | 81-2 (Z.) | analog Beispiel 1 | 167-9 (Z.) | analog Beispiel A4 |

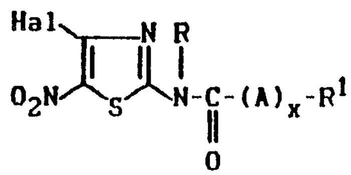

(I)

| | Endprodukte | | | | | Ausgangsverbindung | |
|---|---|---|---|---|---|---|---|
| Verb. Nr. | Hal | -R | $-(A)_x-R^1$ | Schmp. (°C) (umkrist. aus) | Herstellung | Schmp. (°C) | Herstellung |
| 56 | Cl | 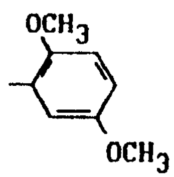 | $-CH_3$ | 221 | analog Beispiel 1 | 153-4 (Z.) | analog Beispiel A4 |
| 57 | Cl | 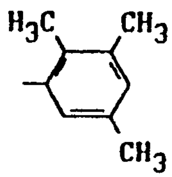 | $-CH_3$ | 226 | analog Beispiel 1 | 208-9 (Z.) | analog Beispiel A4 |
| 58 | Cl | 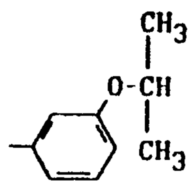 | $-CH_3$ | 151 | analog Beispiel 1 | 48-9 | analog Beispiel A4 |
| 59 | Cl | 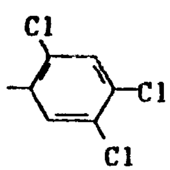 | $-CH_3$ | 187-8 | analog Beispiel 1 | 191-2 (Z.) | analog Beispiel A4 |

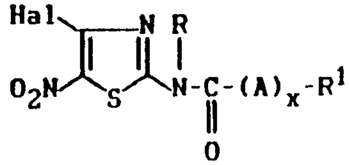 (I)

| Verb. Nr. | Endprodukte Hal | -R | $-(A)_x-R^1$ | Schmp.(°C) (umkrist. aus) | Herstellung | Ausgangsverbindung Schmp.(°C) | Herstellung |
|---|---|---|---|---|---|---|---|
| 60 | Cl | 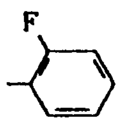 | $-CH_3$ | 166 | analog Beispiel 1 | 157-8 (Z.) | analog Beispiel A4 |
| 61 | Cl | 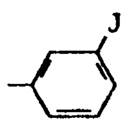 | $-CH_3$ | 147 | analog Beispiel 1 | 158-9 (Z.) | analog Beispiel A4 |
| 62 | Cl | 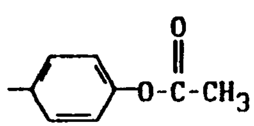 | $-CH_3$ | 197 | siehe Beispiel 13 | 174-6 (Z.) | analog Beispiel A4 |
| 63 | Cl | 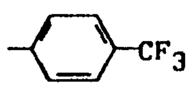 | $-CH_3$ | 190-1 | analog Beispiel 1 | 202-3 (Z.) | analog Beispiel A4 |
| 64 | Cl | 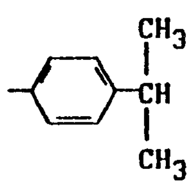 | $-CH_3$ | 148 (Z.) | analog Beispiel 1 | 161-2 (Z.) | analog Beispiel A4 |

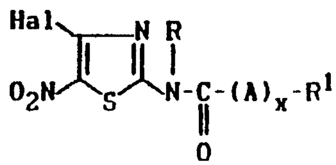

(I)

| Verb. Nr. | Endprodukte Hal | -R | $-(A)_x-R^1$ | Schmp. (°C) (umkrist. aus) | Herstellung | Ausgangsverbindung Schmp. (°C) | Herstellung |
|---|---|---|---|---|---|---|---|
| 65 | Cl | 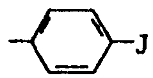 | $-CH_3$ | 260 (Z.) | analog Beispiel 1 | 165-6 (Z.) | analog Beispiel A4 |
| 66 | Cl | 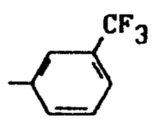 | $-CH_3$ | 160 | analog Beispiel 1 | 121-2 | analog Beispiel A4 |
| 67 | Cl | 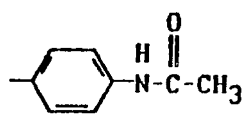 | $-CH_3$ | 201 (Z.) | analog Beispiel 1 | 226-7 | analog Beispiel A4 |
| 68 | Cl | 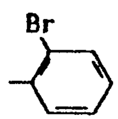 | $-CH_3$ | 122-3 | analog Beispiel 1 | 157-8 (Z.) | analog Beispiel A4 |
| 69 | Cl | 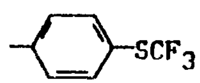 | $-CH_3$ | 130-1 | analog Beispiel 1 | 126 (Z.) | analog Beispiel A4 |
| 70 | Cl | $-CH_3$ | 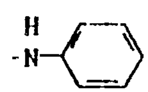 | 225 (Z.) | siehe Beispiel 4 | 212 (Z.) | siehe Beispiel A8 |

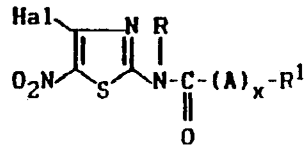

(I)

| Verb. Nr. | Endprodukte Hal | -R | $-(A)_x-R^1$ | Schmp. (°C) (umkrist. aus) | Herstellung | Ausgangsverbindung Schmp. (°C) | Herstellung |
|---|---|---|---|---|---|---|---|
| 71 | Cl | 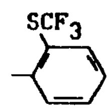 | $-CH_3$ | 135 | analog Beispiel 1 | 126-7 (Z.) | analog Beispiel A4 |
| 72 | Cl | 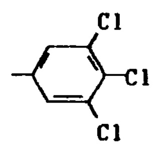 | $-CH_3$ | 142 | analog Beispiel 1 | 221 (Z.) | analog Beispiel A4 |
| 73 | Cl | 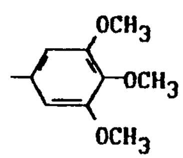 | $-CH_3$ | 226 (Z.) | analog Beispiel 1 | 177-8 (Z.) | analog Beispiel A4 |
| 74 | Cl | 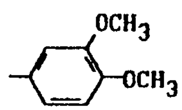 | $-CH_3$ | 111 | analog Beispiel 1 | 201 (Z.) | analog Beispiel A4 |
| 75 | Cl | 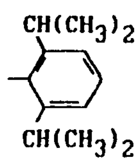 | $-CH_3$ | 114 | analog Beispiel 1 | 228 (Z.) | analog Beispiel A4 |

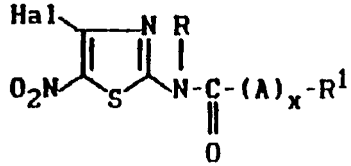

(I)

| Verb. Nr. | Endprodukte Hal | -R | $-(A)_x-R^1$ | Schmp. (°C) (umkrist. aus) | Herstellung | Ausgangsverbindung Schmp. (°C) | Herstellung |
|---|---|---|---|---|---|---|---|
| 76 | Cl | 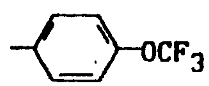 | $-CH_3$ | 157 | analog Beispiel 1 | 121 (Z.) | analog Beispiel A4 |
| 77 | Cl | 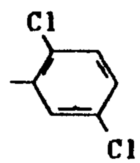 | $-CH_3$ | 164 | analog Beispiel 1 | 136-8 (Z.) | analog Beispiel A4 |
| 78 | Cl | 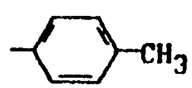 | $-CH_3$ | 232-3 (Z.) | analog Beispiel 1 | 194 (Z.) | analog Beispiel A4 |
| 79 | Cl | 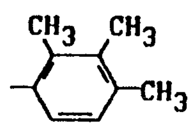 | $-CH_3$ | 172-3 | analog Beispiel 1 | 216-7 (Z.) | analog Beispiel A4 |
| 80 | Cl | 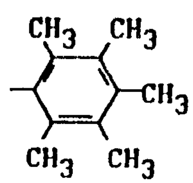 | $-CH_3$ | 260 (Z.) | analog Beispiel 1 | 243-4 (Z.) | analog Beispiel A4 |

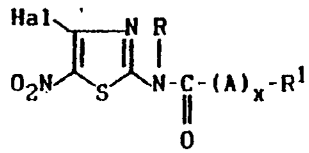

(I)

| Verb. Nr. | Endprodukte Hal | -R | $-(A)_x-R^1$ | Schmp.(°C) (umkrist. aus) | Herstellung | Ausgangsverbindung Schmp.(°C) | Herstellung |
|---|---|---|---|---|---|---|---|
| 81 | Cl | 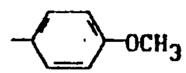 | $-CH_3$ | 169-70 | analog Beispiel 1 | 193 (Z.) | analog Beispiel A4 |
| 82 | Cl | 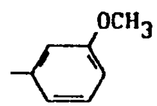 | $-CH_3$ | 197 | analog Beispiel 1 | 175-6 (Z.) | analog Beispiel A4 |
| 83 | Cl | 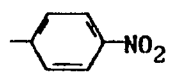 | $-CH_3$ | 142 | analog Beispiel 1 | 262-3 (Z.) | analog Beispiel A4 |
| 84 | Cl | 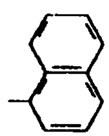 | $-CH_3$ | 227-8 (Z.) | analog Beispiel 1 | 178-9 (Z.) | analog Beispiel A4 |
| 85 | Cl | 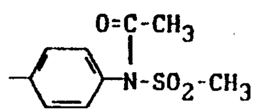 | $-CH_3$ | 246-7 (Z.) | siehe Beispiel 11 | 203 (Z.) | analog Beispiel A4 |
| 86 | Cl | 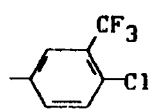 | $-CH_3$ | 224 (Z.) | analog Beispiel 1 | 124-5 (Z.) | analog Beispiel A4 |

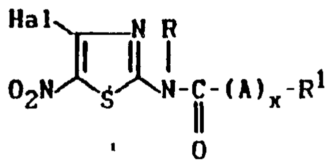

(I)

| Verb. Nr. | Endprodukte: Hal | -R | $-(A)_x-R^1$ | Schmp. (°C) (umkrist. aus) | Herstellung | Ausgangsverbindung: Schmp. (°C) | Herstellung |
|---|---|---|---|---|---|---|---|
| 87 | Cl | 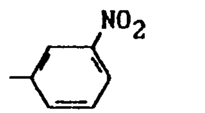 | $-CH_3$ | 213 (Z.) | analog Beispiel 1 | 163-4 (Z.) | analog Beispiel A4 |
| 88 | Cl | 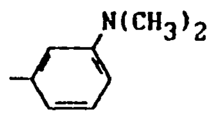 | $-CH_3$ | 226 (Z.) | analog Beispiel 1 | 169-70 (Z.) | analog Beispiel A4 |
| 89 | Cl | 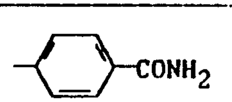 | $-CH_3$ | 154-5 | analog Beispiel 1 | 259 (Z.) | analog Beispiel A4 |
| 90 | Cl | 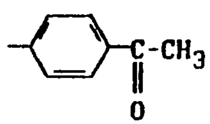 | $-CH_3$ | 187-8 | analog Beispiel 1 | 262-3 (Z.) | analog Beispiel A4 |
| 91 | Cl | 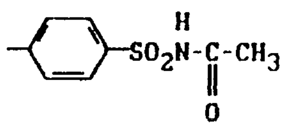 | $-CH_3$ | 244 (Z.) | siehe Beispiel 14 | > 280 | analog Beispiel A4 |
| 92 | Cl | 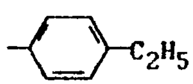 | $-CH_3$ | 177-9 | analog Beispiel 1 | 174-5 (Z.) | analog Beispiel A4 |

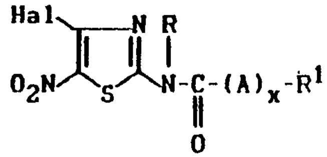

(I)

| Verb. Nr. | Endprodukte Hal | -R | $-(A)_x-R^1$ | Schmp. (°C), (umkrist. aus) | Herstellung | Ausgangsverbindung Schmp. (°C) | Herstellung |
|---|---|---|---|---|---|---|---|
| 93 | Cl | 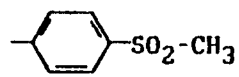 | $-CH_3$ | 255-6 (Z.) | analog Beispiel 1 | 268-9 (Z.) | analog Beispiel A4 |
| 94 | Cl | 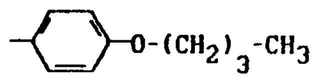 | $-CH_3$ | 88 | analog Beispiel 1 | 154-5 (Z.) | analog Beispiel A4 |
| 95 | Cl | 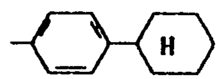 | $-CH_3$ | 155-6 | analog Beispiel 1 | 185-6 (Z.) | analog Beispiel A4 |
| 96 | Cl | $-C_2H_5$ | $-CH_3$ | 109 | analog Beispiel 1 | 167-8 (Z.) | analog Beispiel A8 |
| 97 | Cl | $-CH_2-C_2H_5$ | $-CH_3$ | 120-1 | analog Beispiel 1 | ab 106 (Z.) | analog Beispiel A8 |
| 98 | Cl | $-CH_2-CH_2-OCH_3$ | $-CH_3$ | 115-6 | analog Beispiel 1 | 124-8 (Z.) | analog Beispiel A8 |
| 99 | Cl | $-CH_2-CH_2-Cl$ | $-CH_3$ | 95-6 | analog Beispiel 1 | 143-4 (Z.) | analog Beispiel A8 |

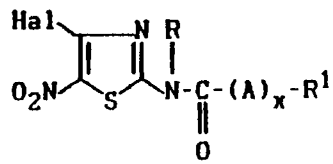

(I)

| Verb. Nr. | Endprodukte Hal | -R | $-(A)_x-R^1$ | Schmp. (°C) (umkrist. auş) | Herstellung | Ausgangsverbindung Schmp. (°C) | Herstellung |
|---|---|---|---|---|---|---|---|
| 100 | Cl | $-CH_2-CH=CH_2$ | $-CH_3$ | 63-4 | analog Beispiel 1 | 130 | analog Beispiel A4 |
| 101 | Cl | 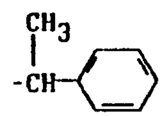 | $-CH_3$ | 117-8 (Z.) | siehe Beispiel 7 | 108 | analog Beispiel A4 |
| 102 | Cl | 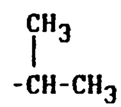 | $-CH_3$ | 131-3 | analog Beispiel 7 | 125-7 (Z.) | analog Beispiel A8 |
| 103 | Cl | 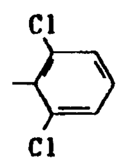 | $-CH_3$ | 95 | siehe Beispiel 15 | 141 | siehe Beispiel A11 |
| 104 | Cl | $-CH_3$ | $-OCH_3$ | 157-8 | siehe Beispiel 9 | 130-1 | siehe Beispiel A9 |
| 105 | Cl | $-CH_3$ | 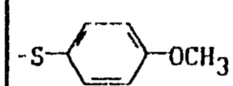 | 183-4 | siehe Beispiel 10 | 130-1 | siehe Beispiel A9 |

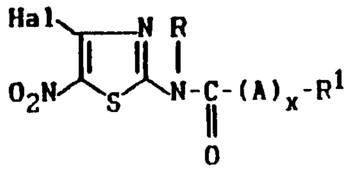 (I)

| Verb. Nr. | Endprodukte: Hal | -R | $-(A)_x-R^1$ | Schmp. (°C) (umkrist. aus) | Herstellung | Ausgangsverbindung: Schmp. (°C) | Herstellung |
|---|---|---|---|---|---|---|---|
| 106 | Cl | $-CH_3$ | 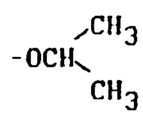 | 116-8 | analog Beispiel 9 | 130-1 | siehe Beispiel A9 |
| 107 | Cl | 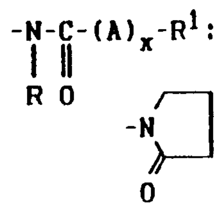 | | 172,5-173 (Cyclohexan) | siehe Beispiel 16 | | |
| 108 | Cl | $-CH_3$ | 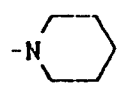 | 168-71 (Z.) | siehe Beispiel 17 und Beispiel 18 | 130-1<br>212 (Z.) | siehe Beispiel A9<br>siehe Beispiel A8 |

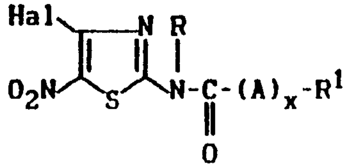

(I)

| Verb. Nr. | Endprodukte Hal | -R | $-(A)_x-R^1$ | Schmp. (°C) (umkrist. aus) | Herstellung | Ausgangsverbindung Schmp. (°C) | Herstellung |
|---|---|---|---|---|---|---|---|
| 109 | Cl | $-CH_3$ | $-NH_2$ | 191-2 | analog Beispiel 8 | 130-1 | siehe Beispiel A9 |
| 110 | Cl | $-CH_3$ | $-S-CH_2-COOC_2H_5$ | 109 | analog Beispiel 10 | 130-1 | siehe Beispiel A9 |
| 111 | Cl | $-CH_3$ | 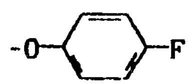 | 199 | analog Beispiel 10 | 130-1 | siehe Beispiel A9 |
| 112 | Cl | $-CH_3$ | 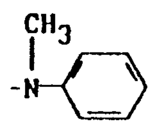 | 165-6 | analog Beispiel 10 | 130-1 | siehe Beispiel A9 |
| 113 | Cl | $-CH_3$ | 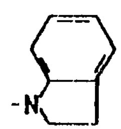 | 181-2 (Z .) | analog Beispiel 10 | 130-1 | siehe Beispiel 9 |

(Z.) = unter Zersetzung

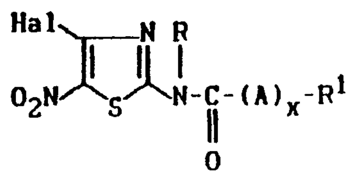

(I)

| Verb. Nr. | Endprodukte Hal | -R | $-(A)_x-R^1$ | Schmp. (°C) (umkrist. aus) | Herstellung | Ausgangsverbindung Schmp. (°C) | Herstellung |
|---|---|---|---|---|---|---|---|
| 114 | Cl | $-CH_3$ | 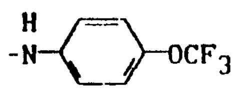 | 209 (Z.) | analog Beispiel 10 | 130-1 | siehe Beispiel A9 |
| 115 | Cl | $-CH_3$ | H<br>$-N-C(CH_3)_3$ | 158-60 (Z.) | analog Beispiel 10 | 130-1 | siehe Beispiel A9 |

Verwendungsbeispiele

Beispiel A

Cochliobolus sativus-Test (Gerste) protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Cochliobolus sativus besprüht. Die Pflanzen verbleiben 48 Stunden bei 20°C und 100 % relativer Luftfeuchtigkeit in einer Inkubationskabine.

Die Pflanzen werden in einem Gewächshaus bei einer Temperatur von ca. 20°C und einer relativen Luftfeuchtigkeit von ca. 80 % aufgestellt.

7 Tage nach der Inokulation erfolgt die Auswertung.

Die Verbindungen, z.B. gemäß den Verbindungsnummern 3, 5, 9, 25, 27, 29, 32 und 35, zeigen bei 0,025 Gew.-% Wirkstoffkonzentration in der Spritzbrühe einen Wirkungsgrad zwischen 70 und 85 %.

Beispiel B

Leptosphaeria nodorum-Test (Weizen) protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Sporensuspension von Leptosphaeria norodum besprüht. Die Pflanzen verbleiben 48 Stunden bei 20°C und 100 % relativer Luftfeuchtigkeit in einer Inkubationskabine.

Die Pflanzen werden in einem Gewächshaus bei einer Temperatur von ca. 15°C und einer relativen Luftfeuchtigkeit von ca. 80 % aufgestellt.

10 Tage nach der Inokulation erfolgt die Auswertung.

Die Verbindungen gemäß den Verbindungsnummern 2, 3, 4, 13, 5, 8, 9, 24 und 34 zeigen bei 0,025 Gew.-% Wirkstoffkonzentration in der Spritzbrühe einen Wirkungsgrad zwischen 70 und 85 %.

Beispiel C

Phytophthora-Test (Tomate) protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung bis zur Tropfnässe. Nach Antrocknen des Spritzbelages werden die Pflanzen mit einer wässrigen Sporensuspension von Phytophthora infestans inokuliert.

Die Pflanzen werden in einer Inkubationskabine mit 100 % relativer Luftfeuchtigkeit und ca. 20 °C aufgestellt.

3 Tage nach der Inokulation erfolgt die Auswertung.

Die Verbindungen gemäß den Verbindungsnummern 10, 13, 17, 16 und 18 zeigen bei einer Wirkstoffkonzentration von 10 ppm einen Wirkungsgrad zwischen 50 und 75 %.

Beispiel D

Zum Nachweis der Wirksamkeit gegen Pilze werden die minimalen Hemm-Konzentrationen (MHK) von erfindungsgemäßen Wirkstoffen bestimmt:

Ein Agar, der aus Bierwürze und Pepton hergestellt wird, wird mit erfindungsgemäßen Wirkstoffen in Konzentrationen von 0,1 mg/l bis 5000 mg/l versetzt. Nach Erstarren des Agars erfolgt Kontaminaton mit Reinkulturen der unten aufgeführten Testorganismen. Nach zweiwöchiger Lagerung bei 28 °C und 60 bis 70 % rel. Luftfeuchtigkeit wird die MHK bestimmt. MHK ist die niedrigste Konzentration an Wirkstoff, bei der keinerlei Bewuchs durch die verwendete Mikrobenart erfolgt.

Z.B. zeigen die Verbindungen der Verbindungsnummer 20, 25, 3, 13, 17, 15, 24, 2, 23, 12, 16 und 26 eine gute Wirksamkeit in diesem Test.

Testorganismen:

Alternaria tenuis
Aspergillus niger
Aureobasidium pullulans
Chaetomium globosum
Cladosporium cladosporioides
Lentinus tigrinus
Penicillium glaucum
Sclerophoma pityophila

**Ansprüche**

1. 2-Acylamino-4-halogen-5-nitrothiazol-Derivate der Formel (I)

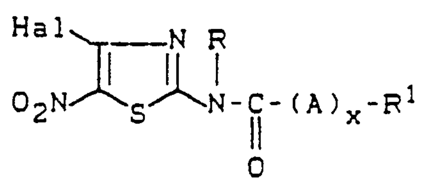

(I)

in welcher
Hal für Halogen steht,
x für eine ganze Zahl 0 oder 1 steht,
A für O, S oder N-$R^2$ steht, worin
$R^2$ für Wasserstoff, Alkyl, Alkenyl, Halogenalkyl, Cyanalkyl, Alkoxycarbonylalkyl oder für jeweils gegebenenfalls ein- bis mehrfach, gleich oder verschieden substituiertes Cycloalkyl, Aryl oder Aralkyl steht,
R für Wasserstoff, Alkyl, Alkenyl oder Alkinyl steht, wobei die vorgenannten Reste jeweils gegebenenfalls ein- bis mehrfach, gleich oder verschieden durch Halogen, Alkoxy, Aryloxy, Alkylmercapto, Arylmercapto und Cyano substituiert sein können, für gegebenenfalls ein-bis mehrfach, gleich oder verschieden durch Alkyl substituiertes Cycloalkyl steht, wobei zusätzlich zu der Alkylsubstitution ein Ring ankondensiert sein kann, für gegebenenfalls ein- bis mehrfach, gleich oder verschieden durch Halogen, Alkyl, Halogenalkyl, Nitro, Alkoxy, Alkylmercapto und Cyan im Arylteil substituiertes Aralkyl steht oder für gegebenenfalls ein- bis mehrfach, gleich oder verschieden durch Halogen, Alkyl, Alkenyl, Alkinyl, Halogenalkyl, Nitro, Alkoxy, Alkylmercapto, Dialkylamino, Carbalkoxy, Carbamoyl, N-Alkylcarbamoyl, N,N-Dialkylcarbamoyl, Halogenalkyloxy, Halogenalkylmercapto, Alkylsulfonylamino, Alkylsulfonyl, Aryl, Aryloxy, Arylmercapto, Acyloxy, Acyl, Sulfamoyl, N-Alkylsulfamoyl, N-Acyl-N-alkylsulfamoyl, N,N-Dialkylsulfamoyl, Aralkyloxy, Aralkylmercapto, Acylamino, Acylaminosulfonyl, Acylalkylamino, Cycloalkyl und Cyan substituiertes Aryl steht und

$R^1$ für Wasserstoff, Alkyl, Alkenyl, Alkinyl, Halogenalkyl, Cyanalkyl, Alkoxycarbonylalkyl oder für jeweils gegebenenfalls ein- bis mehrfach, gleich oder verschieden substituiertes Cycloalkyl, Aryl, Heteroaryl, Aralkyl oder Heteroaralkyl steht oder

R und $R^1$ gemeinsam mit der Gruppierung -N-CO-$(A)_x$-, an der sie stehen, einen Ring bilden, der gegebenenfalls durch ein oder mehrere weitere, gleiche oder verschiedene Heteroatome unterbrochen sein kann und der gegebenenfalls ein-bis mehrfach, gleich oder verschieden substituiert sein kann oder

$R^1$ und $R^2$ gemeinsam mit dem Stickstoffatom, an dem sie stehen, einen Ring bilden, der gegebenenfalls durch ein oder mehrere weitere, gleiche oder verschiedene Heteroatome unterbrochen sein kann und der gegebenenfalls ein- bis mehrfach, gleich oder verschieden substituiert sein kann und gegebenenfalls weitere Ringe ankondensiert sein können,

mit der Ausnahme, daß $R^1$ nicht Wasserstoff ist, wenn A für O oder S steht und ausgenommen die Verbindung 2-Acetylamino-4-iodo-5-nitro-thiazol.

2. 2-Acylamino-4-halogen-5-nitrothiazol-Derivate gemäß Anspruch 1 der Formel (I), in welcher

Hal für Chlor, Brom oder Iod steht,

x für eine ganze Zahl 0 oder 1 steht,

A für O, S oder $NR^2$ steht, wobei

$R^2$ für Wasserstoff, Alkyl mit 1 bis 4 Kohlenstoffatomen, Alkenyl mit 3 bis 5 Kohlenstoffatomen, Halogenalkyl mit 1 bis 3 Kohlenstoffatomen und 1 bis 5 gleichen oder verschiedenen Halogenatomen, für Cyanalkyl mit 1 oder 2 Kohlenstoffatomen im Alkylteil, für Alkoxycarbonylalkyl mit jeweils 1 bis 4 Kohlenstoffatomen im Alkoxy- und Alkylteil, für Cycloalkyl mit 3 bis 6 Kohlenstoffatomen, für gegebenenfalls jeweils ein- bis dreifach, gleich oder verschieden durch Alkyl mit 1 bis 4 Kohlenstoffatomen, Fluor, Chlor, Brom oder Alkoxy mit 1 bis 4 Kohlenstoffatomen substituiertes Phenyl oder Phenylalkyl mit 1 bis 3 Kohlenstoffatomen im Alkylteil steht und

R für Wasserstoff, Alkyl mit 1 bis 12 Kohlenstoffatomen, Alkenyl mit bis zu 12 Kohlenstoffatomen, Alkinyl mit bis zu 12 Kohlenstoffatomen, Halogenalkyl, Halogenalkenyl oder Halogenalkinyl mit jeweils bis zu 8 Kohlenstoffatomen und 1 bis 10, gleichen oder verschiedenen Halogenatomen steht, für Alkoxyalkyl, Alkylmercaptoalkyl oder Cyanalkyl mit 1 bis 4 Kohlenstoffatomen je Alkyl-, Alkoxy-bzw. Alkylthioteil steht, für Phenyloxyalkyl oder Phenylmercaptoalkyl mit 1 bis 4 Kohlenstoffatomen im Alkylteil steht, wobei die Phenylreste gegebenenfalls ein- bis dreifach, gleich oder verschieden durch Fluor, Chlor, Brom oder Alkyl mit 1 bis 4 Kohlenstoffatomen substituiert sein können, für gegebenenfalls ein- bis dreifach, gleich oder verschieden durch Alkyl mit 1 bis 4 Kohlenstoffatomen substituiertes Cycloalkyl mit 3 bis 8 Kohlenstoffatomen steht, wobei der Cycloalkylring zusätzlich einen ankondensierten Ring enthalten kann, für gegebenenfalls ein- bis fünffach, gleich oder verschieden durch Halogen, Alkyl mit 1 bis 4 Kohlenstoffatomen, Halogenalkyl mit 1 bis 4 Kohlenstoffatomen und 1 bis 8 gleichen oder verschiedenen Halogenatomen, Nitro, Cyano, Alkoxy mit 1 bis 4 Kohlenstoffatomen oder Alkylmercapto mit 1 bis 4 Kohlenstoffatomen substituiertes Phenylalkyl mit 1 bis 4 Kohlenstoffatomen im Alkylteil steht, wobei der Alkylteil gegebenenfalls einen weiteren Phenylrest als Substituenten enthält, der gegebenenfalls, wie vorher angegeben, substituiert sein kann, oder für gegebenenfalls ein- bis fünffach, gleich oder verschieden durch Halogen, Nitro, Alkenyl oder Alkinyl mit jeweils bis zu 5 Kohlenstoffatomen, Alkyl, Alkoxy, Alkylmercapto, Carbalkoxy, Alkylsulfonylamino, Alkylsulfonyl, Sulfamoyl, N-Alkylsulfamoyl, N,N-Dialkylsulfamoyl, Dialkylamino, Carbamoyl, N-Alkylcarbamoyl, N,N-Dialkylcarbamoyl mit jeweils 1 bis 4 Kohlenstoffatomen je Alkylrest, Halogenalkyl, Halogenalkoxy, Halogenalkylmercapto mit jeweils 1 bis 4 Kohlenstoffatomen und 1 bis 8 gleichen oder verschiedenen Halogenatomen je aufgezähltem Rest, Phenyl, Phenoxy, Phenylmercapto, Acyloxy mit 1 bis 3 Kohlenstoffatomen, Acyl mit 1 bis 3 Kohlenstoffatomen, Phenylalkyloxy mit 1 bis 3 Kohlenstoffatomen im Alkylteil, Phenylalkylmercapto mit 1 bis 3 Kohlenstoffatomen, Acylamino mit 1 bis 3 Kohlenstoffatomen, Acylalkylamino, N-Acyl-alkylsulfamoyl oder Acylaminosulfonyl mit jeweils 1 bis 3 Kohlenstoffatomen je Acyl- und Alkylrest, Cycloalkyl mit 4 bis 6 Kohlenstoffatomen und Cyan substituiertes Phenyl oder für Naphthyl steht,

$R^1$ für Wasserstoff, Alkyl mit 1 bis 6 Kohlenstoffatomen, Alkenyl oder Alkinyl mit jeweils 3 bis 5 Kohlenstoffatomen, Halogenalkyl mit 1 bis 5 Kohlenstoffatomen und 1 bis 8 gleichen oder verschiedenen Halogenatomen, Cyanalkyl mit 1 oder 2 Kohlenstoffatomen im Alkylteil, Alkoxycarbonylalkyl mit jeweils 1 bis 4 Kohlenstoffatomen im Alkoxy- und Alkylteil, für gegebenenfalls ein- bis dreifach durch Alkyl mit 1 bis 3 Kohlenstoffatomen substituiertes Cycloalkyl mit 3 bis 6 Kohlenstoffatomen, für gegebenenfalls jeweils ein- bis dreifach, gleich oder verschieden durch Alkyl mit 1 bis 4 Kohlenstoffatomen, Fluor, Chlor, Brom oder Alkoxy, Halogenalkyl oder Halogenalkoxy mit je 1 bis 4 Kohlenstoffatomen und gegebenenfalls mit 1 bis 5, gleichen oder verschiedenen Halogenatomen substituiertes Phenyl oder Phenylalkyl mit 1 bis 3 Kohlenstoffatomen im Alkylteil oder für Heterocyclyl oder Heterocyclylalkyl mit 1 bis 4 Kohlenstoffatomen im Alkylteil und 5 bis 7 Ringgliedern im Heterocyclylteil, der ein bis drei, gleiche oder verschiedene Heteroatome, enthalten kann und denen weitere Ringe ankondensiert sein können, steht, wobei die Ringe jeweils gegebenenfalls gleich oder verschieden, ein- bis dreifach substituiert sein können durch Halogen, Alkyl mit 1 bis 4 Kohlenstoffatomen und Alkoxy mit 1 bis 3 Kohlenstoffatomen, oder

R und $R^1$ gemeinsam mit der Gruppierung $-N-CO-(A)_x-$, an der sie stehen, einen Ring mit 5 bis 7 Ringgliedern bilden, der gegebenenfalls ein oder zwei weitere Stickstoff- und/oder Sauerstoffatome enthalten kann und gegebenenfalls ein- bis dreifach durch Alkyl mit 1 bis 4 Kohlenstoffatomen substituiert sein kann, oder

$R^1$ und $R^2$ gemeinsam mit dem Stickstoffatom, an dem sie stehen, einen Ring mit 5 bis 7 Ringgliedern bilden, der gegebenenfalls ein oder zwei weitere Stickstoff- und/oder Sauerstoffatome enthalten kann und gegebenenfalls ein- bis dreifach durch Alkyl mit 1 bis 4 Kohlenstoffatomen substituiert sein kann und gegebenenfalls weitere Ringe ankondensiert sein können,

mit der Ausnahme, daß $R^1$ nicht Wasserstoff ist, wenn A für O oder S steht und ausgenommen die Verbindung 2-Acetylamino-4-iodo-5-nitrothiazol.

3. 2-Acylamino-4-halogen-5-nitrothiazol-Derivate der Formel (I) gemäß Anspruch 1, in welcher

Hal für Chlor steht,

x für O steht,

R für Wasserstoff, geradkettiges oder verzweigtes Alkyl mit 1 bis 6 Kohlenstoffatomen, geradkettiges oder verzweigtes Alkenyl mit bis zu 6 Kohlenstoffatomen, Halogenalkyl mit 1 bis 4 Kohlenstoffatomen und 1 bis 6 gleichen oder verschiedenen Halogenatomen, Alkoxyalkyl mit jeweils 1 bis 3 Kohlenstoffatomen je Alkoxy- bzw. Alkylteil, ferner für Cyclohexyl, Naphthyl, gegebenenfalls ein- bis dreifach, gleich oder verschieden durch Alkyl mit 1 bis 3 Kohlenstoffatomen und Halogen substituiertes Phenylalkyl mit 1 bis 4 Kohlenstoffatomen im geradkettigen oder verzweigten Alkylteil, oder für gegebenenfalls ein- bis fünffach, gleich oder verschieden durch Alkyl mit 1 bis 3 Kohlenstoffatomen oder gegebenenfalls ein- bis dreifach, gleich oder verschieden durch Halogen, Nitro, Cyclohexyl, Alkyl mit 1 bis 4 Kohlenstoffatomen, Alkoxy mit 1 bis 4 Kohlenstoffatomen, Alkylsulfonyl mit 1 bis 4 Kohlenstoffatomen, Dialkylamino mit 1 bis 4 Kohlenstoffatomen je Alkylteil, Carbamoyl, N- Alkyl- oder N,N-Dialkyl-carbamoyl mit 1 bis 4 Kohlenstoffatomen je Alkylteil, Halogenalkoxy oder Halogenalkylmercapto mit jeweils 1 bis 4 Kohlenstoffatomen und 1 bis 5 gleichen oder verschiedenen Halogenatomen, Acyl oder Acylamino mit jeweils 1 bis 3 Kohlenstoffatomen, Acyloxy mit 1 bis 3 Kohlenstoffatomen, N-Acyl-alkylsulfamoyl oder Acylaminosulfonyl mit jeweils 1 bis 3 Kohlenstoffatomen je Acyl-und Alkylteil substituiertes Phenyl steht und

$R^1$ für Alkyl mit 1 bis 6 Kohlenstoffatomen, Halogenalkyl mit 1 bis 3 Kohlenstoffatomen und 1 bis 5 gleichen oder verschiedenen Halogenatomen, für gegebenenfalls 1- bis 3-fach durch Methyl, Ethyl oder Propyl substituiertes Cyclohexyl, für gegebenenfalls jeweils ein-bis dreifach, gleich oder verschieden durch Alkyl mit 1 bis 3 Kohlenstoffatomen, Fluor, Chlor, Brom, Alkoxy, Halogenalkyl oder Halogenalkoxy mit je 1 bis 3 Kohlenstoffatomen und gegebenenfalls 1 bis 5, gleichen oder verschiedenen Halogenatomen substituiertes Phenyl oder für einen gegebenenfalls ein- bis dreifach, gleich oder verschieden durch Chlor, Methyl, Ethyl, n- oder iso-Propyl substituierten 5- oder 6-gliedrigen Ring mit 1 bis 3 gleichen oder verschiedenen Heteroatomen steht, ausgenommen die Verbindung 2-Acetylamino-4-iodo-5-nitro-thiazol.

4. 2-Acylamino-4-halogen-5-nitrothiazol-Derivate der Formel (I) gemäß Anspruch 1, in welcher

R für Wasserstoff, geradkettiges oder verzweigtes Alkyl mit 1 bis 6 Kohlenstoffatomen, geradkettiges oder verzweigtes Alkenyl mit bis zu 6 Kohlenstoffatomen, Halogenalkyl mit 1 bis 4 Kohlenstoffatomen und 1 bis 6 gleichen oder verschiedenen Halogenatomen, Alkoxyalkyl mit jeweils 1 bis 3 Kohlenstoffatomen je Alkoxy- bzw. Alkylteil, ferner für Cyclohexyl, Naphthyl, gegebenenfalls ein- bis dreifach, gleich oder verschieden durch Alkyl mit 1 bis 3 Kohlenstoffatomen und Halogen substituiertes Phenylalkyl mit 1 bis 4 Kohlenstoffatomen im geradkettigen oder verzweigten Alkylteil, oder für gegebenenfalls ein- bis fünffach, gleich oder verschieden durch Alkyl mit 1 bis 3 Kohlenstoffatomen oder gegebenenfalls ein- bis dreifach, gleich oder verschieden durch Halogen, Nitro, Cyclohexyl, Alkyl mit 1 bis 4 Kohlenstoffatomen, Alkoxy mit 1 bis 4 Kohlenstoffatomen, Alkylsulfonyl mit 1 bis 4 Kohlenstoffatomen, Dialkylamino mit 1 bis 4 Kohlenstoffatomen je Alkylteil, Carbamoyl, N-Alkyl- oder N,N-Dialkyl-carbamoyl mit 1 bis 4 Kohlenstoffatomen je Alkylteil, Halogenalkoxy oder Halogenalkylmercapto mit jeweils 1 bis 4 Kohlenstoffatomen und 1 bis 5 gleichen oder verschiedenen Halogenatomen. Acyl oder Acylamino mit jeweils 1 bis 3 Kohlenstoffato men, Acyloxy mit 1 bis 3 Kohlenstoffatomen, N-Acyl-alkylsulfamoyl oder Acylaminosulfonyl mit jeweils 1 bis 3 Kohlenstoffatomen je Acyl-und Alkylteil substituiertes Phenyl steht und

x für 1 steht,

A für O oder S steht und

$R^1$ für Alkyl mit 1 bis 4 Kohlenstoffatomen, für gegebenenfalls ein- bis dreifach, gleich oder verschieden

durch Halogen, Alkoxy, Alkyl, Halogenalkyl und Halogenalkoxy mit jeweils 1 bis 3 Kohlenstoffatomen und gegebenenfalls mit 1 bis 5, gleichen oder verschiedenen Halogenatomen substituiertes Phenyl oder für einen 5- oder 6-gliedrigen Ring mit 1 oder 2 gleichen oder verschiedenen Heteroatomen steht

oder

R und $R^1$ mit der Gruppierung $-N-CO-(A)_x-$, an der sie stehen, einen 5- oder 6-gliedrigen Ring bilden, der gegebenenfalls 1- bis 3-fach, gleich oder verschieden substituiert sein kann durch Halogen und Alkyl mit 1 bis 4 Kohlenstoffatomen.

5. 2-Acylamino-4-halogen-5-nitrothiazol-Derivate der Formel (I) gemäß Anspruch 1, in welcher

R für Wasserstoff, geradkettiges oder verzweigtes Alkyl mit 1 bis 6 Kohlenstoffatomen, geradkettiges oder verzweigtes Alkenyl mit bis zu 6 Kohlenstoffatomen, Halogenalkyl mit 1 bis 4 Kohlenstoffatomen und 1 bis 6 gleichen oder verschiedenen Halogenatomen, Alkoxyalkyl mit jeweils 1 bis 3 Kohlenstoffatomen je Alkoxy- bzw. Alkylteil, ferner für Cyclohexyl, Naphthyl, gegebenenfalls ein- bis dreifach, gleich oder verschieden durch Alkyl mit 1 bis 3 Kohlenstoffatomen und Halogen substituiertes Phenylalkyl mit 1 bis 4 Kohlenstoffatomen im geradkettigen oder verzweigten Alkylteil, oder für gegebenenfalls ein- bis fünffach, gleich oder verschieden durch Alkyl mit 1 bis 3 Kohlenstoffatomen oder gegebenenfalls ein- bis dreifach, gleich oder verschieden durch Halogen, Nitro, Cyclohexyl, Alkyl mit 1 bis 4 Kohlenstoffatomen, Alkoxy mit 1 bis 4 Kohlenstoffatomen, Alkylsulfonyl mit 1 bis 4 Kohlenstoffatomen, Dialkylamino mit 1 bis 4 Kohlenstoffatomen je Alkylteil, Carbamoyl, N-Alkyl- oder N,N-Dialkyl-carbamoyl mit 1 bis 4 Kohlenstoffatomen je Alkylteil, Halogenalkoxy oder Halogenalkylmercapto mit jeweils 1 bis 4 Kohlenstoffatomen und 1 bis 5 gleichen oder verschiedenen Halogenatomen, Acyl oder Acylamino mit jeweils 1 bis 3 Kohlenstoffatomen, Acyloxy mit 1 bis 3 Kohlenstoffatomen, N-Acyl-alkylsulfamoyl oder Acylaminosulfonyl mit jeweils 1 bis 3 Kohlenstoffatomen je Acyl-und Alkylteil substituiertes Phenyl steht und

x für 1 steht,

A für $NR^2$ steht,

$R^1$ für Wasserstoff, Alkyl mit 1 bis 6 Kohlenstoffatomen, Halogenalkyl mit 1 bis 3 Kohlenstoffatomen und 1 bis 5 gleichen oder verschiedenen Halogenatomen, für gegebenenfalls 1- bis 3-fach durch Methyl, Ethyl oder Propyl substituiertes Cyclohexyl, für gegebenenfalls jeweils ein-bis dreifach, gleich oder verschieden durch Alkyl mit 1 bis 3 Kohlenstoffatomen, Fluor, Chlor, Brom, Alkoxy, Halogenalkyl oder Halogenalkoxy mit je 1 bis 3 Kohlenstoffatomen und gegebenenfalls 1 bis 5, gleichen oder verschiedenen Halogenatomen substituiertes Phenyl oder für einen gegebenenfalls ein- bis dreifach, gleich oder verschieden durch Chlor, Methyl, Ethyl, n- oder iso-Propyl substituierten 5- oder 6-gliedrigen Ring mit 1 bis 3 gleichen oder verschiedenen Heteroatomen, wie Stickstoff, Schwefel oder Sauerstoff, steht, ausgenommen die Verbindung 2-Acetylamino-4-iodo-5-nitro-thiazol und

$R^2$ für Wasserstoff, Alkyl mit 1 bis 4 Kohlenstoffatomen, Alkenyl mit 3 bis 5 Kohlenstoffatomen, Halogenalkyl mit 1 bis 3 Kohlenstoffatomen und 1 bis 5 gleichen oder verschiedenen Halogenatomen, für Cyanalkyl mit 1 oder 2 Kohlenstoffatomen im Alkylteil, für Cycloalkyl mit 3 bis 6 Kohlenstoffatomen, für gegebenenfalls jeweils ein- bis dreifach, gleich oder verschieden durch Alkyl mit 1 bis 4 Kohlenstoffatomen, Fluor, Chlor, Brom oder Alkoxy mit 1 bis 4 Kohlenstoffatomen substituiertes Phenyl oder Phenylalkyl mit 1 bis 3 Kohlenstoffatomen im Alkylteil steht und

$R^1$ und $R^2$ gemeinsam mit dem Stickstoffatom, an dem sie stehen, einen Ring mit 5 bis 7 Ringgliedern bilden, der gegebenenfalls ein oder zwei weitere Stickstoff- und/oder Sauerstoffatome enthalten kann und gegebenenfalls ein- bis dreifach durch Alkyl mit 1 bis 4 Kohlenstoffatomen substituiert sein kann und weitere Ringe ankondensiert sein können.

6. Verfahren zur Herstellung von 2-Acylamino-4-halogen-5-nitrothiazol-Derivaten der Formel (I)

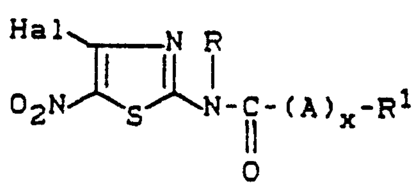

(I)

in welcher

Hal für Halogen steht,

x für eine ganze Zahl 0 oder 1 steht,

A für O, S oder $N-R^2$ steht, worin

$R^2$ für Wasserstoff, Alkyl, Alkenyl, Halogenalkyl, Cyanalkyl, Alkoxycarbonylalkyl oder für jeweils gegebenenfalls ein- bis mehrfach, gleich oder verschieden substituiertes Cycloalkyl, Aryl oder Aralkyl steht,
R für Wasserstoff, Alkyl, Alkenyl oder Alkinyl steht, wobei die vorgenannten Reste jeweils gegebenenfalls ein- bis mehrfach, gleich oder verschieden durch Halogen, Alkoxy, Aryloxy, Alkylmercapto, Arylmercapto und Cyano substituiert sein können, für gegebenenfalls ein-bis mehrfach, gleich oder verschieden durch Alkyl substituiertes Cycloalkyl steht, wobei zusätzlich zu der Alkylsubstitution ein Ring ankondensiert sein kann, für gegebenenfalls ein- bis mehrfach, gleich oder verschieden durch Halogen, Alkyl, Halogenalkyl, Nitro, Alkoxy, Alkylmercapto und Cyan im Arylteil substituiertes Aralkyl steht oder für gegebenenfalls ein- bis mehrfach, gleich oder verschieden durch Halogen, Alkyl, Alkenyl, Alki nyl, Halogenalkyl, Nitro, Alkoxy, Alkylmercapto, Dialkylamino, Carbalkoxy, Carbamoyl, N-Alkylcarbamoyl, N,N-Dialkylcarbamoyl, Halogenalkyloxy, Halogenalkylmercapto, Alkylsulfonylamino, Alkylsulfonyl, Aryl, Aryloxy, Arylmercapto, Acyloxy, Acyl, Sulfamoyl, N-Alkylsulfamoyl, N-Acyl-N-alkylsulfamoyl, N,N-Dialkylsulfamoyl, Aralkyloxy, Aralkylmercapto, Acylamino, Acylaminosulfonyl, Acylalkylamino, Cycloalkyl und Cyan substituiertes Aryl steht und
$R^1$ für Wasserstoff, Alkyl, Alkenyl, Alkinyl, Halogenalkyl, Cyanalkyl, Alkoxycarbonylalkyl oder für jeweils gegebenenfalls ein- bis mehrfach, gleich oder verschieden substituiertes Cycloalkyl, Aryl, Heteroaryl, Aralkyl oder Heteroaralkyl steht oder
R und $R^1$ gemeinsam mit der Gruppierung $-N-CO-(A)_x-$, an der sie stehen, einen Ring bilden, der gegebenenfalls durch ein oder mehrere weitere, gleiche oder verschiedene Heteroatome unterbrochen sein kann und der gegebenenfalls ein-bis mehrfach, gleich oder verschieden substituiert sein kann oder
$R^1$ und $R^2$ gemeinsam mit dem Stickstoffatom, an dem sie stehen, einen Ring bilden, der gegebenenfalls durch ein oder mehrere weitere, gleiche oder verschiedene Heteroatome unterbrochen sein kann und der gegebenenfalls ein- bis mehrfach, gleich oder verschieden substituiert sein kann und gegebenenfalls weitere Ringe ankondensiert sein können,
mit der Ausnahme, daß $R^1$ nicht Wasserstoff ist, wenn A für O oder S steht, dadurch gekennzeichnet, daß
a) 2,4-Dihalogen-5-nitrothiazole der Formel (II)

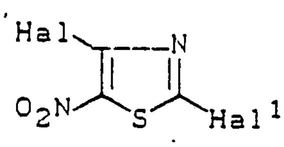

(II)

in welcher
$Hal^1$ für Halogen steht,
Hal die oben angegebene Bedeutung hat und
Hal und $Hal^1$ gleich oder verschieden sein können,
mit Nucleophilen der Formel (III)

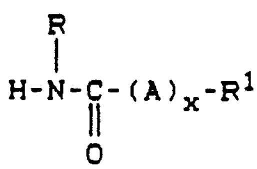

(III)

in welcher
A, x, R und $R^1$ die oben angegebene Bedeutung haben, oder deren Metallsalze gegebenenfalls in Gegenwart von Säurebindemitteln und in Gegenwart von Verdünnungsmitteln umsetzt,
oder
b) 2-Amino-4-halogen-5-nitrothiazol-Derivate der Formel (IV)

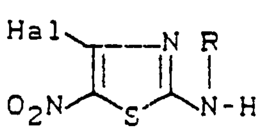

(IV)

in welcher

Hal und R die oben angegebenen Bedeutungen haben, mit Halogencarbonyl-Verbindungen der Formel (V)

$Hal^3$- C -$(A)_x$-R' (V)
||
O

in welcher
$Hal^3$ für Halogen steht und
A, X und R' die oben angegebene Bedeutung haben,
gegebenenfalls in Gegenwart von Säurebindemitteln und in Gegenwart von Verdünnungsmitteln umsetzt, oder

c) für den Fall, daß x für 0 steht und R' die oben angegebene Bedeutung ausgenommen Wasserstoff hat,
2-Amino-4-halogen-5-nitrothiazol-Derivate der Formel (IV) mit Carbonsäureanhydriden der Formel (VI)

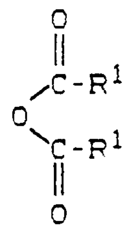
(VI)

in welcher
R' die oben angegebene Bedeutung ausgenommen Wasserstoff hat,
gegebenenfalls in Gegenwart von Verdünnungsmitteln umsetzt,
oder

d) für den Fall, daß x für 1 und A für NH steht, 2-Amino-4-halogen-5-nitrothiazol-Derivate der Formel (IV) mit Isocyanaten der Formel (VII)
O=C=N-R' (VII)
in welcher
R' die oben angegebene Bedeutung ausgenommen Wasserstoff hat,
gegebenenfalls in Gegenwart von Verdünnungsmitteln umsetzt,
oder

e) für den Fall, daß x für 1 steht und A die angegebene Bedeutung hat,
4-Halogen-2-halogencarbonylamino-5-nitrothiazol-Derivate der Formel (VIII)

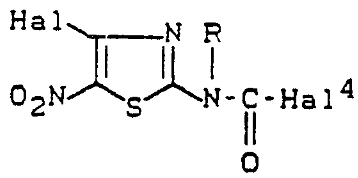
(VIII)

in welcher
$Hal^4$ für Halogen steht,
Hal die oben angegebene Bedeutung hat und
Hal und $Hal^4$ gleich oder verschieden sein können und
R die oben angegebene Bedeutung ausgenommen Wasserstoff hat,
mit Nucleophilen der Formel (IX)
H-$(A)_x$-R' (IX)
in welcher
x für 1 steht und
R' und A die oben angegebene Bedeutung hat,
gegebenenfalls in Gegenwart von Säurebindemitteln sowie in Gegenwart von Verdünnungsmitteln umsetzt.

7. Verwendung von 2-Acylamino-4-halogen-5-nitro-thiazol-Derivaten der Formel (I)

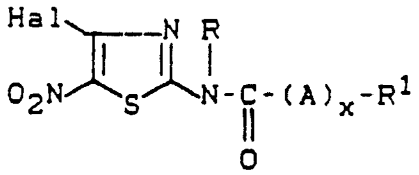

(I)

in welcher
Hal für Halogen steht,
x für eine ganze Zahl 0 oder 1 steht,
A für O, S oder N-$R^2$ steht, worin
$R^2$ für Wasserstoff, Alkyl, Alkenyl, Halogenalkyl, Cyanalkyl, Alkoxycarbonylalkyl oder für jeweils gegebenenfalls ein- bis mehrfach, gleich oder verschieden substituiertes Cycloalkyl, Aryl oder Aralkyl steht,
R für Wasserstoff, Alkyl, Alkenyl oder Alkinyl steht, wobei die vorgenannten Reste jeweils gegebenenfalls ein- bis mehrfach, gleich oder verschieden durch Halogen, Alkoxy, Aryloxy, Alkylmercapto, Arylmercapto und Cyano substituiert sein können, für gegebenenfalls ein-bis mehrfach, gleich oder verschieden durch Alkyl substituiertes Cycloalkyl steht, wobei zusätzlich zu der Alkylsubstitution ein Ring ankondensiert sein kann, für gegebenenfalls ein-bis mehrfach, gleich oder verschieden durch Halogen, Alkyl, Halogenalkyl, Nitro, Alkoxy, Alkylmercapto und Cyan im Arylteil substituiertes Aralkyl steht oder für gegebenenfalls ein- bis mehrfach, gleich oder verschieden durch Halogen, Alkyl, Alkenyl, Alkinyl, Halogenalkyl, Nitro, Alkoxy, Alkylmercapto, Dialkylamino, Carbalkoxy, Carbamoyl, N-Alkylcarbamoyl, N,N-Dialkylcarbamoyl, Halogenalkyloxy, Halogenalkylmercapto, Alkylsulfonylamino, Alkylsulfonyl, Aryl, Aryloxy, Arylmercapto, Acyloxy, Acyl, Sulfamoyl, N-Alkylsul famoyl, N-Acyl-N-alkylsulfamoyl, N,N-Dialkylsulfamoyl, Aralkyloxy, Aralkylmercapto, Acylamino, Acylaminosulfonyl, Acylalkylamino, Cycloalkyl und Cyan substituiertes Aryl steht und
$R^1$ für Wasserstoff, Alkyl, Alkenyl, Alkinyl, Halogenalkyl, Cyanalkyl, Alkoxycarbonylalkyl oder für jeweils gegebenenfalls ein- bis mehrfach, gleich oder verschieden substituiertes Cycloalkyl, Aryl, Heteroaryl, Aralkyl oder Heteroaralkyl steht oder
R und $R^1$ gemeinsam mit der Gruppierung $-N-CO-(A)_x-$, an der sie stehen, einen Ring bilden, der gegebenenfalls durch ein oder mehrere weitere, gleiche oder verschiedene Heteroatome unterbrochen sein kann und der gegebenenfalls ein-bis mehrfach, gleich oder verschieden substituiert sein kann oder
$R^1$ und $R^2$ gemeinsam mit dem Stickstoffatom, an dem sie stehen, einen Ring bilden, der gegebenenfalls durch ein oder mehrere weitere, gleiche oder verschiedene Heteroatome unterbrochen sein kann und der gegebenenfalls ein- bis mehrfach, gleich oder verschieden substituiert sein kann und weitere Ringe ankondensiert sein können,
mit der Ausnahme, daß $R^1$ nicht Wasserstoff ist, wenn A für O oder S steht und ausgenommen die Verbindung 2-Acetylamino-4-iodo-5-nitro-thiazol, nach dem Anspruch 6 zur Bekämpfung von Schädlingen.

8. Schädlingsbekämpfungsmittel, gekennzeichnet durch einen Gehalt an mindestens einem 2-Acylamino-4-halogen-5-nitro-thiazol-Derivat der Formel (I) nach den Ansprüchen 6 und 7.

9. Verfahren zur Bekämpfung von Schädlingen, dadurch gekennzeichnet, daß man 2-Acylamino-4-halogen-5-nitro-thiazol-Derivate der Formel (I) nach den Ansprüchen 6 und 7 auf Schädlinge und/oder ihren Lebensraum einwirken läßt.

10. Verfahren zur Herstellung von Schädlingsbekämpfungsmittel, dadurch gekennzeichnet, daß man 2-Acylamino-4-halogen-5-nitro-thiazol-Derivate der Formel (I) nach den Ansprüchen 6 und 7 mit Streckmitteln und/oder oberflächenaktiven Mitteln vermischt.

11. 4-Halogen-2-halogencarbonylamino-5-nitro-thiazol-Derivate der Formel (VIII)

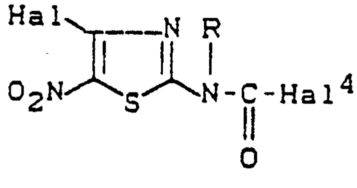

(VIII)

in welcher
R für Wasserstoff, Alkyl, Alkenyl oder Alkinyl steht, wobei die vorgenannten Reste jeweils gegebenenfalls ein- bis mehrfach, gleich oder verschieden durch Halogen, Alkoxy, Aryloxy, Alkylmercapto, Arylmercapto

und Cyano substituiert sein können, für gegebenenfalls ein-bis mehrfach, gleich oder verschieden durch Alkyl substituiertes Cycloalkyl steht, wobei zusätzlich zu der Alkylsubstitution ein Ring ankondensiert sein kann, für gegebenenfalls ein- bis mehrfach, gleich oder verschieden durch Halogen, Alkyl, Halogenalkyl, Nitro, Alkoxy, Alkylmercapto und Cyan im Arylteil substituiertes Aralkyl steht oder für gegebenenfalls ein- bis mehrfach, gleich oder verschieden durch Halogen, Alkyl, Alkenyl, Alkinyl, Halogenalkyl, Nitro, Alkoxy, Alkylmercapto, Dialkylamino, Carbalkoxy, Carbamoyl, N-Alkylcarbamoyl, N,N-Dialkylcarbamoyl, Halogenalkyloxy, Halogenalkylmercapto, Alkylsulfonylamino, Alkylsulfonyl, Aryl, Aryloxy, Arylmercapto, Acyloxy, Acyl, Sulfamoyl, N-Alkylsulfamoyl, N-Acyl-N-alkylsulfamoyl, N,N-Dialkylsulfamoyl, Aralkyloxy, Aralkylmercapto, Acylamino, Acylaminosulfonyl, Acylalkylamino, Cycloalkyl und Cyan substituiertes Aryl steht und
Hal und $Hal^4$ für gleiches oder verschiedenes Halogen stehen.

12. Verfahren zur Herstellung von 4-Halogen-2-halogen-carbonylamino-5-nitro-thiazol-Derivaten der Formel (VIII)

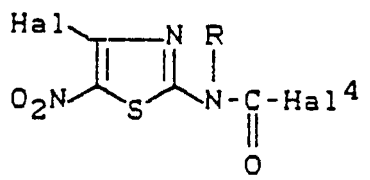

(VIII)

in welcher
R für Wasserstoff, Alkyl, Alkenyl oder Alkinyl steht, wobei die vorgenannten Reste jeweils gegebenenfalls ein- bis mehrfach, gleich oder verschieden durch Halogen, Alkoxy, Aryloxy, Alkylmercapto, Arylmercapto und Cyano substituiert sein können, für gegebenenfalls ein-bis mehrfach, gleich oder verschieden durch Alkyl substituiertes Cycloalkyl steht, wobei zusätzlich zu der Alkylsubstitution ein Ring ankondensiert sein kann, für gegebenenfalls ein- bis mehrfach, gleich oder verschieden durch Halogen, Alkyl, Halogenalkyl, Nitro, Alkoxy, Alkylmercapto und Cyan im Arylteil substituiertes Aralkyl steht oder für gegebenenfalls ein- bis mehrfach, gleich oder verschieden durch Halogen, Alkyl, Alkenyl, Alkinyl, Halogenalkyl, Nitro, Alkoxy, Alkylmercapto, Dialkylamino, Carbalkoxy, Carbamoyl, N-Alkylcarbamoyl, N,N-Dialkylcarbamoyl, Halogenalkyloxy, Halogenalkylmercapto, Alkylsulfonylamino, Alkylsulfonyl, Aryl, Aryloxy, Arylmercapto, Acyloxy, Acyl, Sulfamoyl, N-Alkylsulfamoyl, N-Acyl-N-alkylsulfamoyl, N,N-Dialkylsulfamoyl, Aralkyloxy, Aralkylmercapto, Acylamino, Acylaminosulfonyl, Acylalkylamino, Cycloalkyl und Cyan substituiertes Aryl steht und
Hal und $Hal^4$ für gleiches oder verschiedenes Halogen stehen,
dadurch gekennzeichnet, daß man 2-Amino-4-halogen-5-nitro-thiazol-Derivate der Formel (IV)

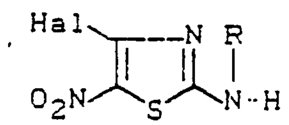

(IV)

in welcher
Hal und R die oben angegebenen Bedeutungen haben,
mit der Ausnahme, daß R nicht Wasserstoff ist, mit Carbonyldihalogeniden der Formel (XI)
$O=C(Hal^4)_2$ (XI)
in welcher
$Hal^4$ die oben angegebene Bedeutung hat, umsetzt.

13. Verwendung von 4-Halogen-2-halogencarbonylamino-5-nitro-thiazol-Derivaten der Formel (VIII) gemäß Ansprüchen 11 und 12 als Ausgangsverbindungen für hochaktive Verbindungen.

Europäisches Patentamt

# EUROPÄISCHER RECHERCHENBERICHT

Nummer der Anmeldung

EP 90110451.3

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int Cl 5) |
|---|---|---|---|
| A | CHEMICAL ABSTRACTS, Band 107, Nr. 21, 23. November 1987, Columbus, Ohio, USA KAKEN PHARMACEUTICAL CO.; YASHIMA CHEMICAL INDUSTRIAL CO. "Preparation of thiazolyl benzoylureas as insecticides" Seite 763, Spalte 2, Zusammenfassung-Nr. 198 306y & Jpn. Kokai Tokkyo Koho JP 62 36 369 (87 36 369) -- | 1,6-13 | C 07 D 277/58<br>C 07 D 277/587<br>C 07 D 417/12<br>A 01 N 43/78 |
| A | FR - A1 - 2 435 905 (AGENCE NATIONALE DE VALORISATION DE LA RECHERCHE) * Anspruch 1; Seite 1, Zeilen 3-12 * -- | 1,6-13 | |
| A | EP - A1 - 0 031 563 (THE WELLCOME FOUNDATION LIMITED) * Zusammenfassung * ---- | 1,6-13 | |

| RECHERCHIERTE SACHGEBIETE (Int Cl 5) |
|---|
| C 07 D 277/00<br>C 07 D 417/00 |

Der vorliegende Recherchenbericht wurde für alle Patentansprüche erstellt

| Recherchenort | Abschlußdatum der Recherche | Prüfer |
|---|---|---|
| WIEN | 25-07-1990 | BRUS |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 62